(19) Europäisches Patentamt
European Patent Office
Office européen des brevets

(11) EP 0 471 358 B1

(12) **EUROPEAN PATENT SPECIFICATION**

(45) Date of publication and mention of the grant of the patent: **08.05.1996 Bulletin 1996/19**

(21) Application number: **91113652.1**

(22) Date of filing: **14.08.1991**

(51) Int. Cl.$^6$: **C07D 491/22**, C07D 491/20, C07D 491/147, A61K 31/435 // (C07D491/22, 311:00, 221:00, 221:00, 209:00)

(54) **Fluoroethylcamptothecin derivatives**

Fluoräthylcamptothecinderivate

Dérivés de fluoréthylcamptothecin

(84) Designated Contracting States:
**BE CH DE ES FR GB IT LI NL SE**

(30) Priority: **14.08.1990 JP 215214/90**

(43) Date of publication of application:
**19.02.1992 Bulletin 1992/08**

(73) Proprietor: **KYORIN PHARMACEUTICAL CO., LTD.**
**Chiyoda-ku Tokyo (JP)**

(72) Inventors:
- **Yoshikazu, Asahina**
  **Shimotsuga-gun, Tochigi-ken (JP)**
- **Kikoh, Obi**
  **Shimotsuga-gun, Tochigi-ken (JP)**
- **Yasuo, Oomori**
  **Shimotsuga-gun, Tochigi-ken (JP)**
- **Takashi, Okazaki**
  **Shimotsuga-gun, Tochigi-ken (JP)**

(74) Representative: **VOSSIUS & PARTNER**
**Postfach 86 07 67**
**81634 München (DE)**

(56) References cited:
**EP-A- 0 220 601** **EP-A- 0 325 247**

- **CHEMICAL ABSTRACTS, vol. 108, No. 19, May 9, 1988, Columbus, Ohio, USA YAKULT HONSHA CO. "Preparation of camptothecin derivatives as antitumor agents" page 681, column 2, abstract -No. 167 768s**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 471 358 B1

Printed by Rank Xerox (UK) Business Services
2.11.6/3.4

## Description

The present invention relates to compounds having antineoplastic activities, and a process of production thereof.

Camptothecin is a pentacyclic alkaloid which is isolated from bark, roots, fruits, leaves, etc. of Camptotheca acuminata (Wall, et al.: J. Am. Chem. Soc., 88, 3888-3890 (1966)), and is known to exhibit antineoplastic activities by inhibiting synthesis of nucleic acid (Lown, et al.: Biochem. Pharmacol., 29, 905-915 (1980)). The development of the camptothecin as a pharmaceutical was interrupted because of its toxicity found in clinical tests in the United States.

EP-A-325 247 relates to camptothecin derivates carrying one or more substituents in 9-, 10-, 11- and/or 12-position in addition to a lower alkyl group in 7-position.

Since then, the derivatives of camptothecin are being investigated throughout the world for achieving alleviation of the toxicity and increase of the activity of the compound. Nevertheless, no derivative has been reported yet which gives a satisfactory clinical effect.

Most of the derivatives of camptothecin reported until now are those having an ethyl group as the alkyl group at the 4-position of the lactone ring. An exception is the one reported by Sugasawa, et al. (J. Med. Chem., 19, 675-679 (1975)), who reported that the derivatives having a substituent such as allyl, propargyl, and benzyl exhibit the activities, but are not improved in pharmacokinetic aspects such as toxicity and absorbability.

Under such a circumstance, the inventors of the present invention made comprehensive investigation to find a camptothecin derivative which exhibits higher activities with less toxicity, and is improved in pharmacokinetic aspects such as absorbability. As the result, it was found that the derivatives having a 2-fluoroethyl group as the alkyl group at the 4-position of the lactone ring has characteristics surpassing the camptothecin, and the present invention has been accomplished.

The present invention intends to provide a camptothecin derivative which exhibits higher activities with less toxicity, and is improved in pharmacokinetic aspects such as absorbability.

The present invention provides a compound represented by the general formula (I) below and a salt thereof:

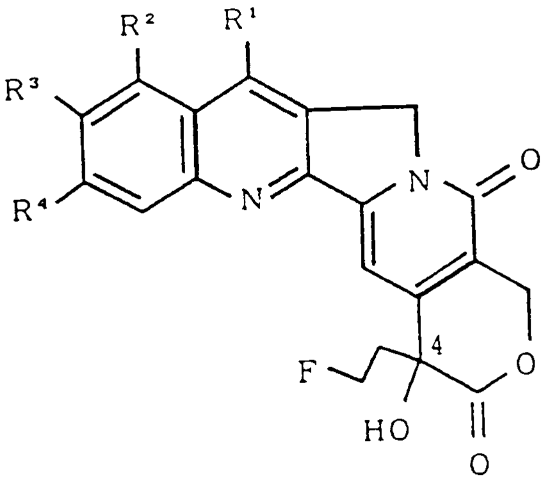

(I)

where $R^1$ is hydrogen, $C_1$-$C_6$ alkyl, hydroxymethyl, acyloxymethyl, and formyl; $R^2$, $R^3$, and $R^4$ are independently hydrogen, hydroxyl, $C_1$-$C_6$ alkyl, $C_2$-$C_6$ alkenyl, $C_2$-$C_6$ alkynyl, $C_1$-$C_6$ alkoxy, halogen, amino, $C_2$-$C_7$ acylamino, $C_1$-$C_6$ alkylamino, $NR^5R^6$ [in which $R^5$ and $R^6$ are independently $C_1$-$C_6$ alkyl or may form a five-membered or six-membered ring together with a nitrogen atom bonded thereto or further together with an atom of O, S, or N-$R^7$ ($R^7$ being hydrogen, $C_1$-$C_6$ alkyl or an amino-protecting group) as a ring constituent, and a ring-constituting carbon may be substituted by $C_1$-$C_3$ alkyl, amino, or aminomethyl], phenyl, naphthyl, or pyridyl; or $R^1$ and $R^2$ may form together a bridged structure represented by $-(CH_2)_m-Z-(CH_2)_n-$ [in which Z is O, S, CH-$R^8$ ($R^8$ being hydrogen or $C_1$-$C_6$ alkyl), or N-$R^{7'}$ ($R^{7'}$ being hydrogen, $C_1$-$C_6$ alkyl, or an amino-protecting group); and m and n are respectively 0, 1, or 2 provided that m and n are not simultaneously zero.].

The present invention also provides a process for producing the compound represented by the aforementioned Formula (I).

In the definition of the compound represented by Formula (I), the groups of alkyl, alkenyl, and alkynyl of $R^1$, $R^2$, $R^3$, $R^4$, $R^7$, and $R^8$, and a corresponding portion of a combination of these group together with another functional group or

substituent have one to six carbons. As the alkyl groups, particularly preferred are methyl and ethyl. The protecting group for the amino group includes formyl, acetyl, trityl, t-butoxycarbonyl, p-methoxybenzyloxycarbonyl, and the like.

More specifically, $R^1$, $R^2$, $R^3$, and $R^4$ include methyl, ethyl, hydroxyl, methoxyl, fluoro, chloro, bromo, nitro, amino, dimethylamino, 3-amino-1-pyrrolidinyl, 3-aminomethyl-1-pyrrolidinyl, 1-piperazinyl, 3-methyl-1-piperazinyl, 4-methyl-1-piperazinyl, 4-morpholinyl, phenyl, 1-naphthyl or 3-pyridyl.

In Formula (I), the asymmetric carbon of the lactone ring has preferably S-configuration in view of its activity. The present invention also relates to a pyranoindolizine derivative represented by the general formula (II):

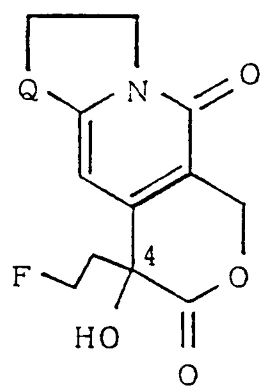

(II)

where Q is C=O or

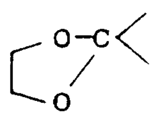

,

a compound, wherein the steric configuration at the 4-position of the compound represented by the general formula (II) is the S-configuration,
a pyranoindolizine derivative represented by the general formula (III) below:

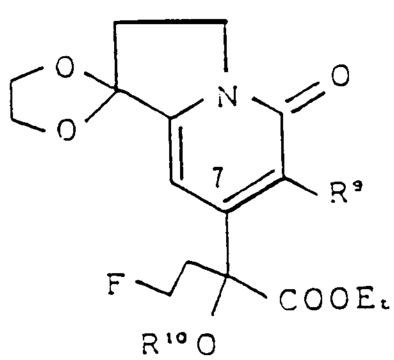

(III)

where $R^9$ is cyano, acetylaminomethyl, or acetoxymethyl; and $R^{10}$ is formyl, acetyl, benzoyl, or (R)-1-(p-toluenesulfonyl)prolyl, and the compound, wherein the steric configuration at the α-position or the acetic acid group at the 7-position of the compound of the general formula (III) is the S-configuration.

The compound of the present invention can be produced, for example, through the process exemplified by the following reaction formula.

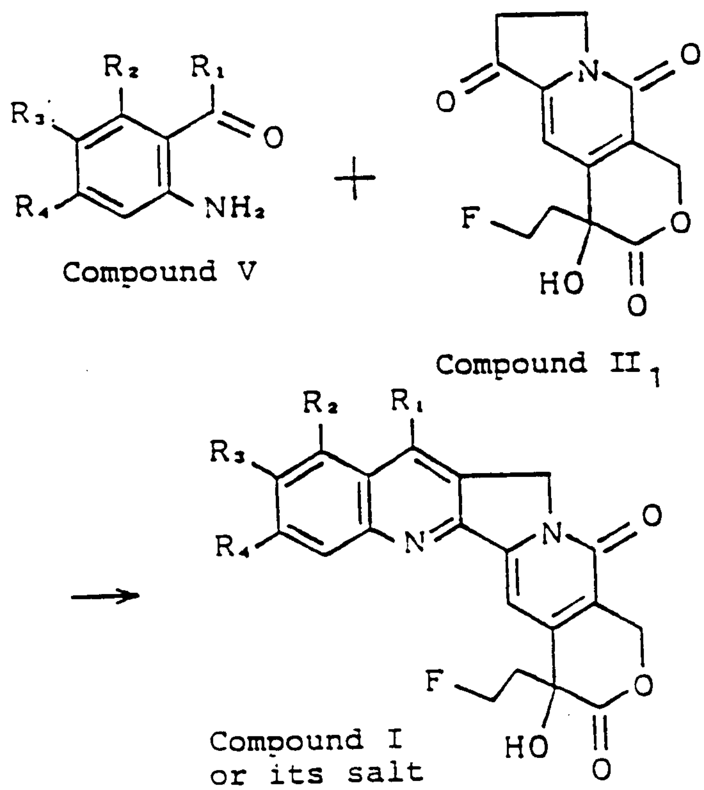

Compound V

Compound $II_1$

Compound I or its salt

where $R^1$, $R^2$, $R^3$, and $R^4$ are as defined above.

An aminoketone compound (V) is condensed with a pyranoindolizine compound ($II_1$) through Friedlander reaction to give a compound (I) (Organic Reactions 28, 37-202, John Wiley & Sons Inc., New York (1982)).

The aminoketone (V) is a known compound or a novel compound readily prepared according to a known method.

The ring-closing condensation reaction of the compound (V) with the compound ($II_1$) is conducted suitably in the presence of an acid or a base at an ordinary or elevated temperature.

Any solvent may be used if it is inert to the reaction, the examples therefor including aromatic hydrocarbons such as benzene, toluene, and xylene; halogenated hydrocarbons such as chloroform, dichloromethane, 1,1-dichloroethane, and 1,2-dichloroethane; ethers such as diethyl ether, diisopropyl ether, tetrahydrofuran, ethylene glycol dimethyl ether, ethylene glycol diethyl ether, and diethylene glycol dimethyl ether; lower alcohols such as methanol, ethanol, propanol, and tertiary butanol; amides such as acetamide, dimethylacetamide, and N,N-dimethylformamide; acetic acid. Among them, preferred are benzene, toluene, and acetic acid.

The acid may be an inorganic acid or an organic acid. The inorganic acid is exemplified by hydrochloric acid, and sulfuric acid. The organic acid is exemplified by sulfonic acids such as methanesulfonic acid, trifluoromethanesulfonic acid, benzenesulfonic acid, and p-toluenesulfonic toluenesulfonic acid; and carboxylic acids such as acetic acid. Preferred are p-toluenesulfonic acid and acetic acid. The acetic acid serves also as the solvent.

The base may be an inorganic base or an organic base. The inorganic base is exemplified by hydroxides, carbonates, and hydrogencarbonates of alkali metals including lithium hydroxide, sodium hydroxide, potassium hydroxide, lithium carbonate, sodium carbonate, potassium carbonate, sodium hydrogencarbonate, and potassium hydrogencarbonate; and sodium hydride as well. The organic base is exemplified by alkali metal alkoxides such as sodium methoxide, sodium ethoxide, and potassium t-butoxide; tertiary alkylamines such as triethylamine, and N,N-diisopropylethylamine; aromatic tertiary amines such as N,N-dimethylaniline, N,N-diethylaniline, and N,N-dimethylaminopyridine; pyridine or 1,8-diazabicycloundecene. Potassium carbonate is preferred as the inorganic base, and triethylamine is preferred as the organic base.

The reaction temperature is in the range of from 20 to 150 °C, preferably from 80 to 120°C.

The reaction time is usually in the range of from 1 to 48 hours. Normally the reaction is completed in the time of from 1 to 24 hours. Typically the reaction is conducted, for example, by heating and refluxing in acetic acid, or by heating

and refluxing in benzene or toluene in the presence of p-toluenesulfonic acid. In the case where $R^1$, $R^2$, $R^3$, $R^4$, etc. are a protected amino group, the protecting group can be removed by a conventional method such as hydrolysis with an acid or an alkali, or reduction reaction.

The compounds having an alkoxyl group can be converted to the corresponding hydroxy compounds by treatment with aluminum chloride or aluminum bromide in an inert solvent such as toluene and benzene, or by heating treatment in a hydrobromic acid solution.

The compounds having an azide group or a nitro group can be converted to the corresponding amino compounds by catalytic reduction by use of platinum or palladium.

The compounds having an amino group can be converted to the corresponding hydroxy compounds by diazotization with sodium nitrite at a low temperature in an acidic medium, and subsequent hydrolysis of the resulting diazonium salt solution.

Furthermore, the compounds having an amino group can be converted to the corresponding halogenated compounds by diazotization as above and subjecting the resulting diazonium salt to Sandmeyer reaction. The Sandmeyer reaction may be conducted with cuprous chloride or cuprous bromide under usually employed conditions.

On the other hand, the pyranoindolizine compound ($II_1$) is a novel compound, which is prepared from Compound (IV) through the process shown below.

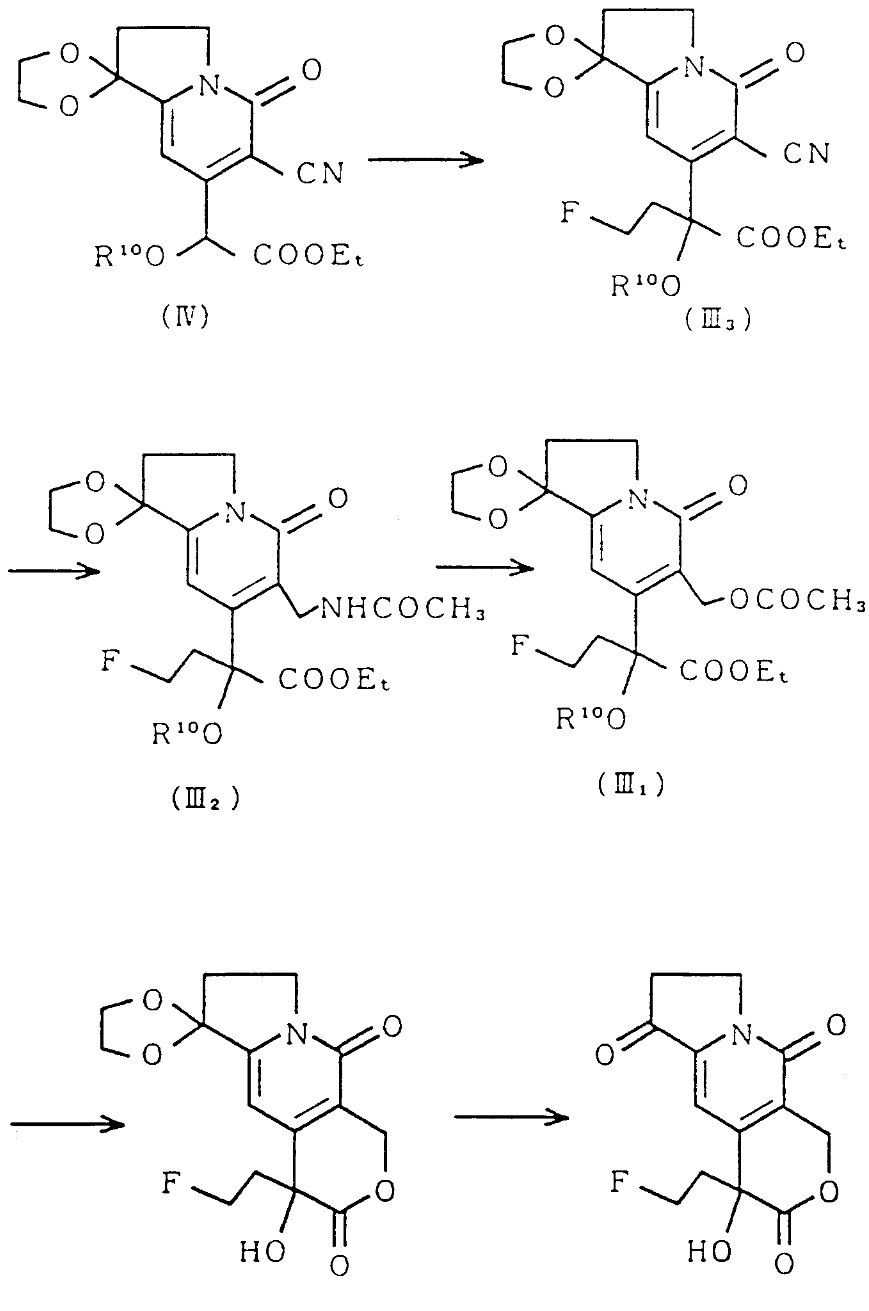

(IV) (III3) (III2) (III1) (II2) (II1)

where $R^{10}$ is formyl, acetyl, benzoyl, or (R)-1-(p-toluenesulfonyl)prolyl.

Compound (IV) is fluoroethylated to give Compound ($III_3$), for example, by reaction with a base such as sodium hydride, and potassium t-butoxide, in a solvent inert to the reaction such as N,N-dimethylformamide and 1,2-dimethoxyethane, and by subsequent addition of a fluoroethyl halide such as fluoroethyl bromide and fluoroethyl iodide or a sulfonate ester of fluoroethanol such as fluoroethyl toluenesulfonate. In particular, in the case where $R^{10}$ is (R)-1-(p-toluenesulfonyl)prolyl, S-configuration of the compound is predominantly obtained. The reaction is conducted usually at a temperature of from -78 to 150°C, preferably from 0 to 80°C for a time of from 10 minutes to 48 hours, preferably from 1 to 24 hours.

The cyano group of Compound ($III_3$) can be reduced by reaction with Raney nickel under hydrogen gas stream, and if necessary with irradiation with a tungsten lamp, to prepare Compound ($III_2$). The reaction is conducted usually at a

temperature of from 10 to 100 °C, preferably from 20 to 60°C for a time of from 10 minutes to 8 hours, preferably from 30 minutes to 5 hours.

Compound ($III_2$) is converted to Compound ($III_1$) by a rearrangement reaction via nitroso compound by reaction with a nitrosation reagent such as sodium nitrite in a mixed solvent of acetic anhydride and acetic acid at a temperature of from 0 to 50°C, preferably from 0 to 30°C for a time of from 30 minutes to 15 hours, preferably from 1 to 5 hours, and subsequent heating of the resulting nitroso compound with stirring at a temperature of from 50 to 120°C, preferably from 60 to 90°C for a time of from 30 minutes to 12 hours, preferably from 1 to 5 hours.

Compound ($III_1$) is converted to Compound ($II_2$) through ring-closing reaction by hydrolysis by an aqueous alkali solution of lithium hydroxide, sodium hydroxide, potassium hydroxide, or the like in a solvent inert to the reaction such as an alcohol like ethanol, and dioxane, and subsequent acid treatment in an acidic solvent employing acetic acid, citric acid or hydrochloric acid. The hydrolysis is conducted at a temperature of from 0 to 50°C, preferably from 20 to 40°C for a time of from 5 minutes to 5 hours, preferably from 10 minutes to 3 hours. The reaction in the acidic solvent is conducted at a temperature of from 0 to 70°C, preferably from 10 to 40°C for a time of 1 to 72 hours, preferably from 12 to 24 hours.

Compound ($II_2$) is converted to Compound ($II_1$) through deketalization by treatment in an acidic solvent employing an acid such as hydrochloric acid, sulfuric acid, and trifluoroacetic acid. The reaction is conducted usually at a temperature of from 0 to 100°C, preferably from 10 to 40°C for a time of from 10 minutes to 24 hours, preferably 10 minutes to 5 hours.

Some of Compounds (I) of the present invention may be converted to a salt thereof by use of an alkali metal or an alkaline earth metal hydroxide, or Compounds (I) having an amino group may be converted to a salt of an inorganic acid such as hydrochloric acid, sulfuric acid, and phosphoric acid, or of an organic acid such as formic acid, and acetic acid, thus physiologically acceptable salts thereof being prepared.

The novel fluoroethylcamptothecin derivatives are useful as pharmaceuticals and intermediate compounds therefor. The pharmaceuticals exhibit antineoplastic activity.

For use as antineoplastic agents, the compounds of the present invention can be administered through various routes. For instance, they can be administered intravenously, intramuscularly, or subcutaneously in a form of injectable solution. An oral route of administration is also applicable.

The present invention is explained in more detail by reference to examples.

EXAMPLE 1

Synthesis of Ethyl 6-cyano-1,1-(ethylenedioxy)-α-(2-fluoroethyl)-α-[(R)-1-(p-toluenesulfonyl)pyrrolidin-2-ylcarbonyloxy]-5-oxo-1,2,3,5-tetrahydroindolizine-7-acetate

920 mg of ethyl 6-cyano-1,1-(ethylenedioxy)-α-[(R)-1-(p-toluenesulfonyl)pyrrolidin-2-ylcarbonyloxy]-5-oxo-1,2,3,5-tetrahydroindolizine-7-acetate was added into a liquid suspension of 80 mg of 60%-sodium hydride in 7 ml of dimethylformamide. The mixture was stirred at room temperature for 1 hour. Into this solution, 2.26 g of 2-fluoroethyl bromide was added, and the mixture was stirred under an argon gas stream at room temperature for 4 hours, and further at 60°C for 2 hours, and still further at 80°C for 2 hours. The reaction mixture was concentrated. The concentration residue was dissolved in 60 ml of chloroform. The solution was washed firstly with water, and then with saturated sodium chloride solution, and was dried over anhydrous sodium sulfate. Subsequently the solution was concentrated. The resulting concentration residue was purified with a silica gel column (eluting solvent: chloroform), thus 750 mg of yellow oily crude product being obtained. This crude product was purified further with the silica gel column (eluting solvent: benzene-ethyl acetate (7:3)) and subsequently with a preparative TLC (developing solvent: benzene-ethyl acetate (7:3)). Thereby 335 mg of the intended product was obtained in a pale yellow resin state.

H-NMR (in $CDCl_3$. δ. ppm)

1.31 (3H. t. $COOCH_2\underline{CH_3}$)

1.60~1.75, 1.92~2.06

(2H. m. 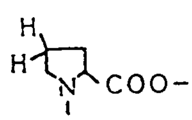 )

1.92~2.18

(2H. m. 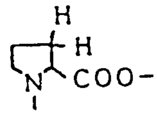 )

2.39~2.45

(5H. m. 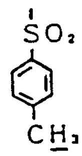 , $C_2$-H)

2.90~3.10

(2H. m. 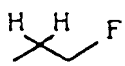 )

3.12~3.25, 3.50~3.60

(2H. m. 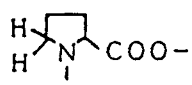 )

4.08~4.12, 4.23~4.26

(4H. m. 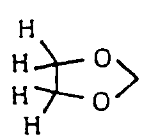 )

4.12~4.21 (2H. m. $C_3$-H)
4.26~4.32 (2H. q. COOC$\underline{H_2}$CH$_3$)
4.43~4.52

(1H. m. 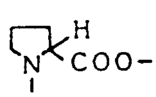 )

4.52~4.72

(2H. m. 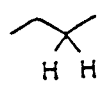 )

6.66 (1H. s. $C_8$-H)

7.34

(2H. d. 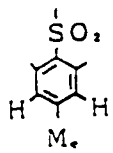 )

7.87

(2H. d. 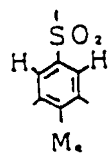 )

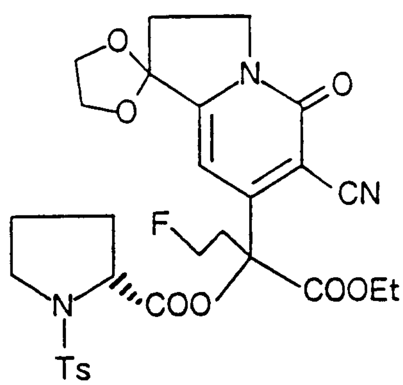

EXAMPLE 2

Synthesis of Ethyl 6-(acetylaminomethyl)-1,1-(ethylenedioxy)-α-(2-fluoroethyl)-α-[(R)-1-(p-toluenesulfonyl)pyrrolidin-2-ylcarbonyloxy]-5-oxo-1,2,3,5-tetrahydroindolizine-7-acetate

280 mg of the compound obtained in Example 1 was added to a mixture of 11 ml of acetic anhydride and 3 ml of acetic acid containing Raney nickel W-2 catalyst prepared from 1120 mg of Raney nickel alloy. The mixture was stirred

under a hydrogen stream at room temperature for 2 hours and further for 1 hour under irradiation with a tungsten lamp. The catalyst was removed from the reaction solution by filtration. The filtrate was concentrated. The concentration residue was dissolved in chloroform. The solution was washed with water, dried over anhydrous sodium sulfate, and concentrated. The concentration residue was purified with a silica gel column (eluting solvent: chloroform-methanol (50:1)). Thus 318 mg of the intended product was obtained in a yellow oil state.

H-NMR (in $CDCl_3$. δ. ppm)

1.27 (3H. t. $COOCH_2C\underline{H}_3$)

1.71~1.73, 1.95~1.98

(2H. m. 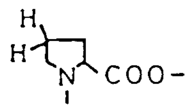 )

1.95

(3H. s. 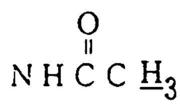 )

1.95~1.98, 2.07~2.15

(2H. m. 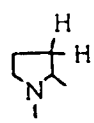 )

2.41~2.48

(5H. m. 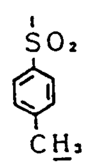 . $C_2$-H)

2.95~3.06

(2H. m. 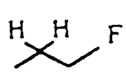 )

3.17~3.28, 3.50~3.55

(2H. m. 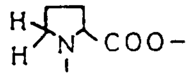 )

4.04~4.33

(9H. m. 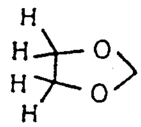 ,
$COOC\underline{H}_2CH_3$, $C_3$-H, 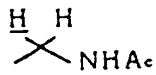 )

4.40~4.44

(1H. m. 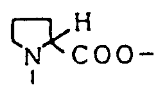 )

4.50~4.70

(2H. m. 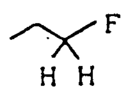 )

4.77~4.87

(1H. m. 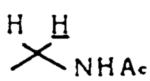 )

6.79 (1H. s. $C_8$-H)
7.11~7.14 (1H. br. -CON$\underline{H}$-)
7.33

(2H. d. 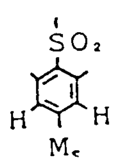 )

7.55

(2H. d. 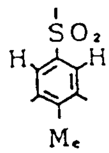 )

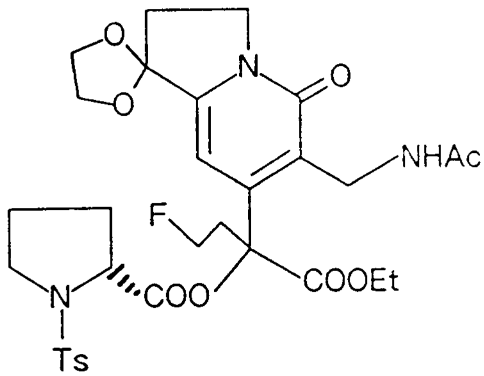

EXAMPLE 3

Synthesis of Ethyl 6-(acetoxymethyl)-1,1-(ethylenedioxy)-α-(2-fluoroethyl)-α-[(R)-1-(p-toluenesulfonyl)pyrrolidin-2-ylcarbonyloxy]-5-oxo-1,2,3,5-tetrahydroindolizine-7-acetate

318 mg of the compound obtained in Example 2 was dissolved in a mixture of 3.2 ml of acetic anhydride and 1 ml of acetic acid. While the solution was cooled with a sodium chloride-ice bath, 140 mg of sodium nitrite was gradually added. The mixture was stirred at that temperature for 5.5 hours with supplemental addition of 140 mg portion of sodium nitrite respectively at the time of 2 hours, and 4 hours of stirring. The stirring was continued further at room temperature for 30 minutes. The insoluble matter was removed from the reaction mixture by filtration. The filtrate was concentrated. To the concentration residue, 8 ml of carbon tetrachloride was added, and the mixture was refluxed by heating for 4 hours. The reaction mixture was diluted with 40 ml of chloroform. The diluted solution was washed with water and then with aqueous saturated sodium chloride solution, dried over anhydrous sodium sulfate, and concentrated. The concentration residue was purified with a silica gel column (eluting solvent: chloroform-methanol (50:1)). Thereby 189 mg of the intended product was obtained in a colorless oil state.

H-NMR (in $CDCl_3$. δ. ppm)
1.35 (3H. t. $COOCH_2C\underline{H_3}$)
1.66~1.72, 1.92~2.01

(2H. m. 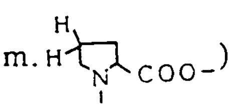 )

1.92~2.03

(2H. m. 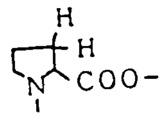 )

2.41 (2H. t. $C_2$-H)

2.45

(3H. s. 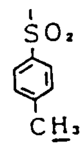 )

2.70~2.80, 3.04~3.17

(2H. m. 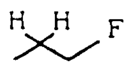 )

3.17~3.24, 3.51~3.59

(2H. m. 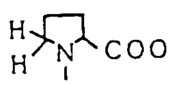 )

4.06~4.28

(8H. m. 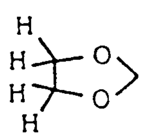 , $COOC\underline{H}_2CH_3$, $C_3$-H)

4.29~4.36

(1H. m. 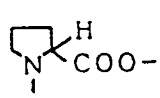 )

4.56

(2H. dt. 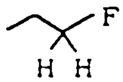 , J = 46. 9Hz)

5.28~5.31

(2H. m. 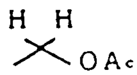 )

6.70 (1H. s. $C_8$-H)

7.35

(2H. d. 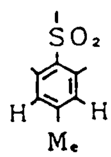 )

7.76

(2H. d. 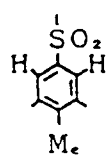 )

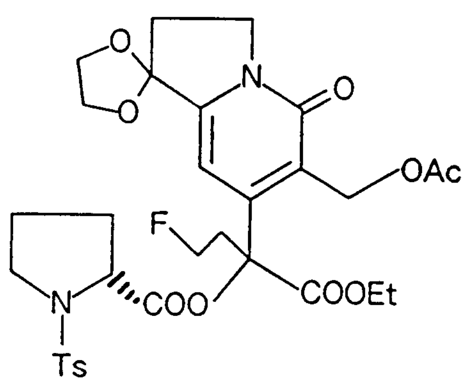

EXAMPLE 4

Synthesis of 6,6-(ethylenedioxy)-4-(2-fluoroethyl)-1,4,7,8-tetrahydro-4-hydroxypyrano[3,4-f]indolizine-3,10 (6H)-dione

3.5 mg of the compound obtained in Example 3 was dissolved in a mixture of 0.1 ml of ethanol and 0.05 ml of water. Thereto 1.0 mg of lithium hydroxide monohydrate was added, and the mixture was stirred at room temperature for 1 hour. The reaction mixture was concentrated. To the concentration residue, 0.3 ml of water, 1.0 ml of methylene chloride, and 0.2 ml of acetic acid were added, and the mixture was stirred at room temperature for 18 hours. The methylene chloride layer was separated, and the water layer was extracted twice with each 1 ml of methylene chloride. The methylene chloride layers were combined and washed with water. The solution was dried over anhydrous sodium sulfate, and concentrated. The concentration residue was purified with preparative TLC (developing solvent: chloroform-methanol (25:1)). Thus 0.89 mg of the intended product was obtained in a white oil state.

H-NMR (in $CDCl_3$. δ. ppm)

2.13~2.15, 2.19~2.22

(2H. m. 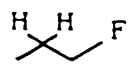 , J=26.8Hz)

2.42 (2H. t. $C_7$-H)

4.08~4.24

(6H. m. 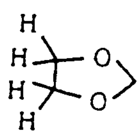 , $C_8$-H)

4.49~4.78

(2H. m. 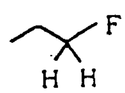 , J=46.4Hz)

5.20, 5.66 (2H. ABq. $C_1$-H, J=16.1Hz)

6.59 (1H. s. $C_5$-H)

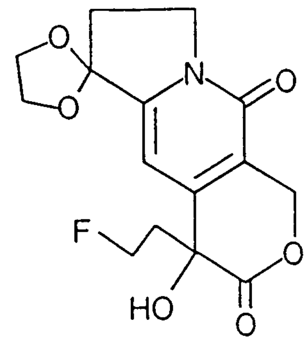

EXAMPLE 5

Synthesis of 4-(2-fluoroethyl)-1,4,7,8-tetrahydro-4-hydroxypyrano[3,4-f]indolizine-3,6,10-trione

0.89 mg of the compound obtained in Example 4 was dissolved in 0.1 ml of aqueous 80%-trifluoroacetic acid. The solution was stirred under an argon gas stream at room temperature for 1 hour. The reaction mixture was concentrated and the resulting concentration residue was purified by preparative TLC (developing solvent: chloroform-methanol (10:1)). Thus 0.55 mg of the intended product was obtained in a red oil state.

H-NMR (in $CDCl_3$. δ. ppm)

2.07~2.30

(2H. m. 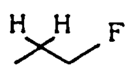 )

2.98 (2H. t. $C_7$-H)

4.31~4.38 (2H. m. $C_8$-H)

4.55~4.65, 4.67~4.77

(2H. m. 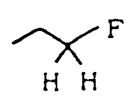 . J=46.9Hz)

5.28, 5.73 (2H. ABq. $C_1$-H, J=17.1Hz)

7.26 (1H. s. $C_5$-H)

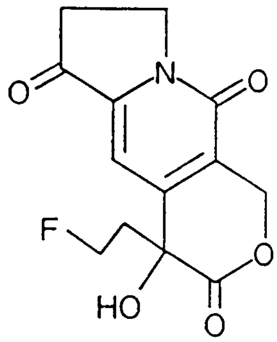

EXAMPLE 6

Synthesis of 11-ethyl-4-(2-fluoroethyl)-4-hydroxy-9-methoxy-1H-pyrano[3',4':6,7]indolizino[1,2-b]quinoline-3,14(4H,12H)-dione

2.03 mg of the compound obtained in Example 5, and 1.12 mg of 2-amino-5-methoxypropiophenone were dissolved in 0.6 ml of toluene. A catalytic amount of p-toluenesulfonic acid were added thereto. The mixture was heated and refluxed for 2 hours. The reaction mixture was concentrated, and the concentration residue was purified by preparative TLC (developing solvent: ethyl acetate-n-hexane (4:1)) to give 1.32 mg of the intended product in a white crystalline state.

Mass m/z=424 ($M^+$)

H-NMR (in $CDCl_3$. δ. ppm)

1.41 (3H. t. $C_{11}$-$CH_2C\underline{H}_3$, J=7.8Hz)

2.22~2.36

(2H. m. 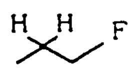 )

3.16 (2H. q. $C_{11}$-$C\underline{H}_2CH_3$, J=7.8Hz)

4.01 (3H. s. $OC\underline{H}_3$)

4.56~4.62, 4.67~4.73, 4.79~4.83

(2H. m. 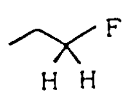 , J=46.9Hz)

5.25 (2H. s. $C_{12}$-H)

5.34, 5.81 (2H. ABq. $C_1$-H)

7.33 (1H. d. $C_{10}$-H, J=2.4Hz)

7.48 (1H. dd. $C_8$-H, J=2.9Hz, 9.3Hz)

7.61 (1H. s. $C_5$-H)

8.14 (1H. d. $C_7$-H, J=9.3Hz)

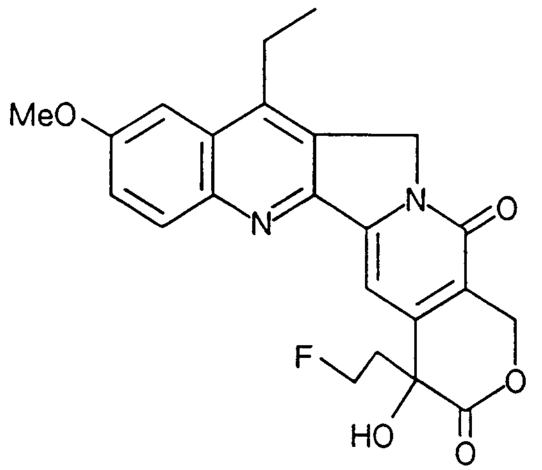

EXAMPLE 7

Synthesis of 11-ethyl-4-(2-fluoroethyl)-4-hydroxy-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.2 mg of 2-aminopropiophenone, 0.5 ml of toluene, and a catalytic amount of p-toluenesulfonic acid were added. The mixture was heated and refluxed for 1.5 hours. The reaction mixture was concentrated, and the concentration residue was purified by preparative TLC (developing solvent: chloroform-methanol (25:1)) to give 2.7 mg of the intended product in a pale yellow crystalline state.

Mass m/z=394 ($M^+$)

H-NMR (in $CDCl_3$. δ. ppm)

1.47 (3H. t. $C_{11}$-$CH_2C\underline{H}_3$, J=7.8Hz)

2.22~2.37

(2 H. m. 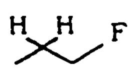 )

3.22 (2H. q. $C_{11}$-$C\underline{H}_2CH_3$)

4.57~4.62, 4.67~4.74, 4.79~4.84

(2 H. m. 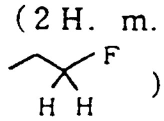 )

5.27 (2H. s. $C_{12}$-H)

5.33, 5.81 (2H. ABq. $C_1$-H, J=16.1Hz)

7.68 (1H. s. $C_5$-H)

7.64~7.71, 7.97~8.34 (1H×2. m×2. $C_8$, $C_9$-H)
8.14, 8.24 (1H×2. dd×2. $C_7$, $C_{10}$-H)

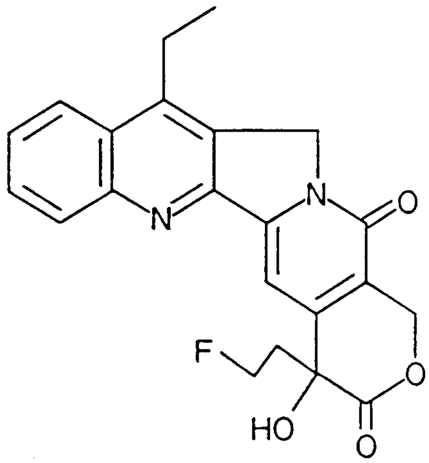

EXAMPLE 8

Synthesis of 11-ethyl-9-fluoro-4-(2-fluoroethyl)-4-hydroxy-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.5 mg of 2-amino-5-fluoropropiophenone, 0,5 ml of toluene, and a catalytic amount of p-toluenesulfonic acid were added. The mixture was heated and refluxed for 1.5 hours. The reaction mixture was concentrated, and the concentration residue was purified by preparative TLC (developing solvent: chloroform-methanol (25:1)) to give 3.3 mg of the intended product in a pale yellow crystalline state.
Mass m/z=412 ($M^+$)
H-NMR (in $CDCl_3$. δ. ppm)
1.41 (3H. t. $C_{11}$-$CH_2C\underline{H_3}$)
2.21~2.35

(2H. m. 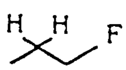 )

3.16 (2H. q. $C_{11}$-$C\underline{H_2}CH_3$)
4.59~4.63, 4.67~4.74, 4.78~4.82

(2H. m.
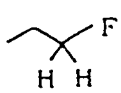 )

5.27 (2H. s. $C_{12}$-H)
5.34, 5.80 (2H. ABq. $C_1$-H)
7.52~7.57 (1H. m. $C_8$-H) 7.65 (1H. s. $C_5$-H)
7.72 (1H. dd. $C_{10}$-H)
8.24 (1H. dd. $C_7$-H)

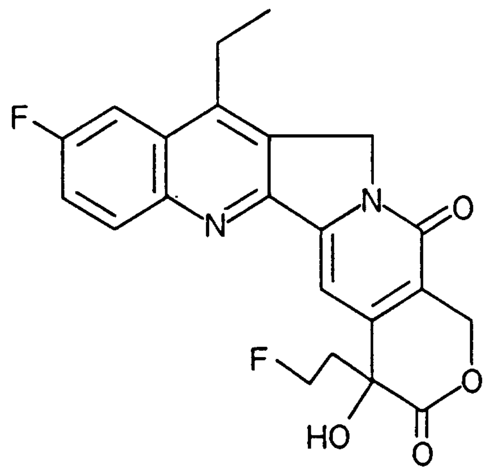

EXAMPLE 9

Synthesis of 9-chloro-11-ethyl-4-(2-fluoroethyl)-4-hydroxy-1H-pyrano[3',4':6,7]indolizino[1,2-b]quinoline-3,14(4H,12H)-dione

5.0 mg of the compound obtained in Example 4 was dissolved in 0,5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.8 mg of 2-amino-5-chloropropiophenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: ethyl acetate) to give 3.2 mg of the intended product in a pale brown crystal state.

H-NMR (in $CDCl_3$, δ, ppm)
1.41 (3H, t, $C_{11}$-$CH_2C\underline{H_3}$)
2.21~2.35 (2H, m, $C\underline{H_2}CH_2F$)
3.17 (3H, q, $C_{11}$-$C\underline{H_2}CH_3$)
4.58~4.82 (2H, m, $CH_2C\underline{H_2}F$)
5.27 (2H, s, $C_{12}$-H)
5.34, 5.80 (2H. ABq, $C_1$-H)
7.66 (1H, s, $C_5$-H)
7.75 (1H, dd, $C_8$-H)
8.08 (1H, d, $C_{10}$-H)
8.16 (1H, d, $C_7$-H)

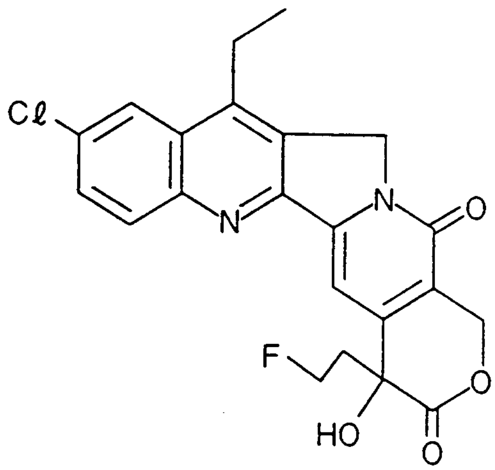

EXAMPLE 10

Synthesis of 9-bromo-11-ethyl-4-(2-fluoroethyl)-4-hydroxy-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.4 mg of 2-amino-5-bromopropiophenone, a catalytic amount of p-toluenesulfonic acid, and 0.5 ml of toluene were added. The mixture was heated and refluxed for 1.5 hour. The reaction mixture was concentrated, and the concentration residue was purified with a silica gel column (eluting solvent: chloroform to chloroform-methanol (10:1)) to give 3.7 mg of the intended product in a red oil state.

H-NMR (in $CDCl_3$, δ, ppm)
1.41 (3H, t, $C_{11}$-$CH_2C\underline{H}_3$)
2.17~2.33 (2H, m, -$C\underline{H}_2CH_2F$)
3.16 (2H, q, $C_{11}$-$C\underline{H}_2CH_3$)
4.59~4.82 (2H, m, $CH_2C\underline{H}_2F$)
5.27 (2H, s, $C_{12}$-H)
5.34, 5.80 (2H, ABq, $C_1$-H)
7.66 (1H, s, $C_5$-H)
7.87 (1H, dd, $C_8$-H)
8.09 (1H, d, $C_7$-H)
8.26 (1H, d, $C_{10}$-H)

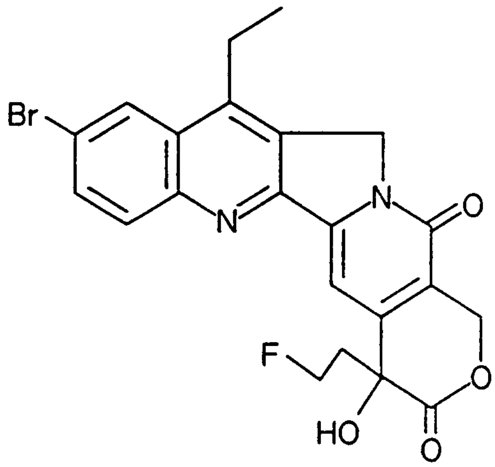

EXAMPLE 11

Synthesis of 11-ethyl-4-(2-fluoroethyl)-4-hydroxy-9-methyl-1H-pyrano[3',4':6,7]indolizino[1,2-b]quinoline-3,14(4H,12H)-dione

5.0 mg of the compound obtained in Example 4 was dissolved in 0,5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.5 mg of 2-amino-5-methylpropiophenone, a catalytic amount of p-toluenesulfonic acid, and 0.5 ml of toluene were added. The mixture was heated and refluxed for 1 hour. The reaction mixture was concentrated, and the concentration residue was purified with a silica gel column (eluting solvent: chloroform to chloroform-methanol (25:1)) to give 3.0 mg of the intended product in a yellow crystal state.

H-NMR (in $CDCl_3$, δ, ppm)
1.40 (3H, t, $C_{11}$-$CH_2C\underline{H_3}$)
2.17~2.34 (2H, m, $C\underline{H_2}CH_2F$)
2.62 (3H, s, $C_9$-$CH_3$)
3.18 (2H, q, $C_{11}$-$C\underline{H_2}CH_3$)
4.57~4.82 (2H, m, $CH_2C\underline{H_2}F$)
5.24 (1H, s, $C_{12}$-H)
5.34, 5.79 (2H, ABq, $C_1$-H)
7.64 (1H, dd, $C_8$-H)
7.64 (1H, s, $C_5$-H)
7.86 (1H, d, $C_{10}$-H)
8.11 (1H, d, $C_7$-H)

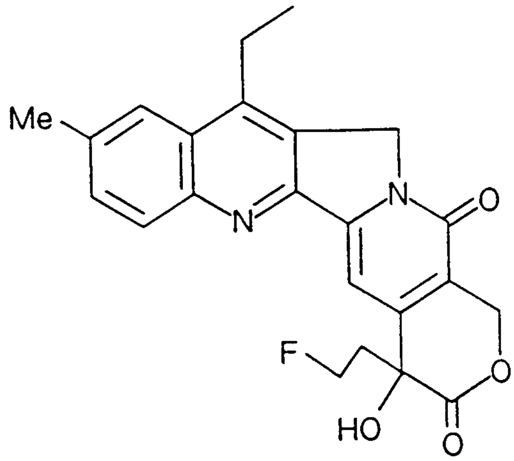

EXAMPLE 12

Synthesis of 11-ethyl-4-(2-fluoroethyl)-4-hydroxy-9-phenyl-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride, The solution was washed with water, and concentrated to dryness. To the resulting concentration residue, 3.4 mg of 2-amino-5-phenylpropiophenone, a catalytic amount of p-toluenesulfonic acid, and 0.5 ml of toluene were added. The mixture was heated and refluxed for 1.5 hours. The reaction mixture was concentrated, and the concentration residue was purified with a silica gel column (eluting solvent: chloroform to chloroform-methanol (25:1)) to give 5.2 mg of the intended product.

H-NMR (in $CDCl_3$. δ. ppm)
1.46 (3H, t, $C_{11}$-$CH_2C\underline{H}_3$)
2.23~2.36 (2H, m, $C\underline{H}_2CH_2F$)
3.27 (2H, q, $C_{11}C\underline{H}_2CH_3$)
4.60~4.84 (2H, m, $CH_2C\underline{H}_2F$)
5.30 (2H, s, $C_{12}$-H)
5.36, 5.82 (2H, ABq, $C_1$-H)
7.48

(1H, t, 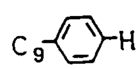 )

7.52~7.57

(2H, t, 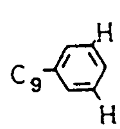 )

7.69 (1H, s, $C_5$-H)
7.75

(2 H, d, 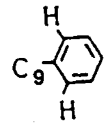 )

8.08 (1H, dd, $C_8$-H)
8.27 (1H, d, $C_{10}$-H)
8.30 (1H, d, $C_7$-H)

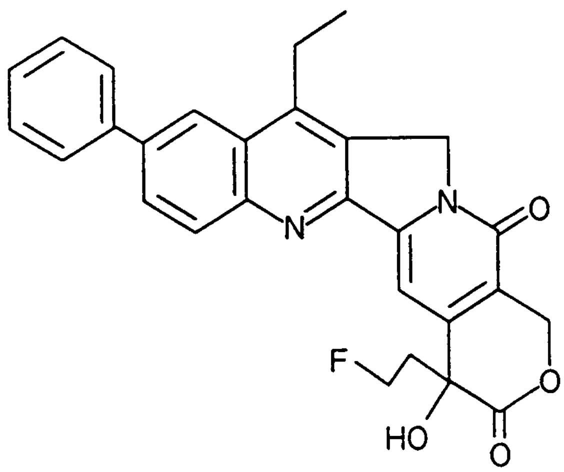

EXAMPLE 13

Synthesis of 11-ethyl-4-(2-fluoroethyl)-4-hydroxy-9-(1-naphthyl)-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.1 mg of 2-amino-5-(1-naphthyl)propiophenone, a catalytic amount of p-toluenesulfonic acid, and 0.5 ml of toluene were added. The mixture was heated and refluxed for 1.5 hours. The reaction mixture was concentrated, and the concentration residue was purified with a silica gel column (eluting solvent: chloroform to chloroform-methanol (25:1)) to give 4.2 mg of the intended product in a yellow oil state.
1.41 (3H, t, $C_{11}$-$CH_2C\underline{H}_3$)
2.24~2.36 (2H, m, $C\underline{H}_2CH_2F$)
3.21 (2H, q, $C_{11}$-$C\underline{H}_2CH_3$)
4.61~4.85 (2H, m, $CH_2C\underline{H}_2F$)
5.31 (2H, s, $C_{12}$-H)
5.37, 5.83 (2H, ABq, $C_1$-H)
7.46~7.63

(4H, m, 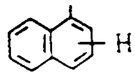 )

7.73 (1H, s, $C_5$-H)
7.91 (1H, dd, $C_8$-H)
7.95~7.99

(3H, m, 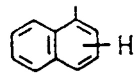 )

8.23 (1H, d, $C_{10}$-H)
8.35 (1H, d, $C_7$-H)

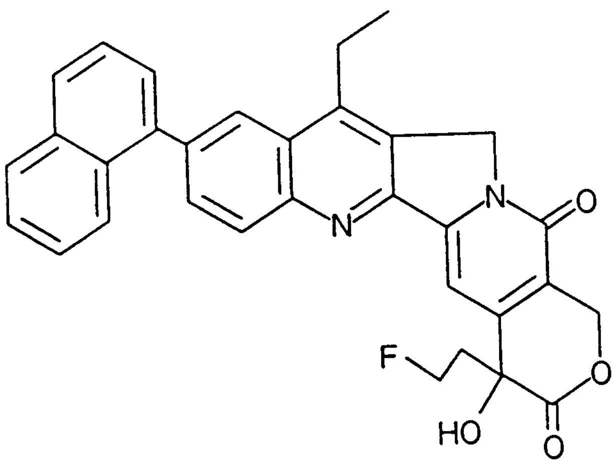

EXAMPLE 14

Synthesis of 11-ethyl-4-(2-fluoroethyl)-4-hydroxy-9-(2-phenylethenyl)-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1.5 hours. The reaction mixture was concentrated, and the resulting concentration residue was dissolved in methylene chloride. The solution was washed with water, and concentrated to dryness. To the resulting concentration residue, 3.6 mg of 2-amino-5-(2-phenylethenyl)propiophenone, a catalytic amount of p-toluenesulfonic acid, and 0.5 ml of toluene were added. The mixture was heated and refluxed for 2 hours. The reaction mixture was concentrated, and the concentration residue was purified with a silica gel column (eluting solvent: chloroform to chloroform-methanol (25:1)) to give 4.6 mg of the intended product.
H-NMR (in $CDCl_3$. δ. ppm)
1.45 (3H, t, $C_{11}$-$CH_2C\underline{H}_3$)
2.21~2.42 (2H, m, $C\underline{H}_2CH_2F$)
3.23 (2H, q, $C_{11}$-$C\underline{H}_2CH_3$)

4.60~4.80 (2H, m, $CH_2C\underline{H_2}F$)
5.27 (2H, s, $C_{12}$-H)
5.35, 5.81 (2H, ABq, $C_1$-H)
7.31~7.35

(3 H, m, CH = CH 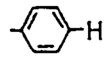 )

7.42

(2 H, t, 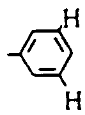 )

7.61

(2 H, d, 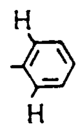 )

7.66 (1H, s, $C_5$-H)
8.06 (1H, d, $C_{10}$-H)
8.10 (1H, dd, $C_8$-H)
8.20 (1H, d, $C_7$-H)

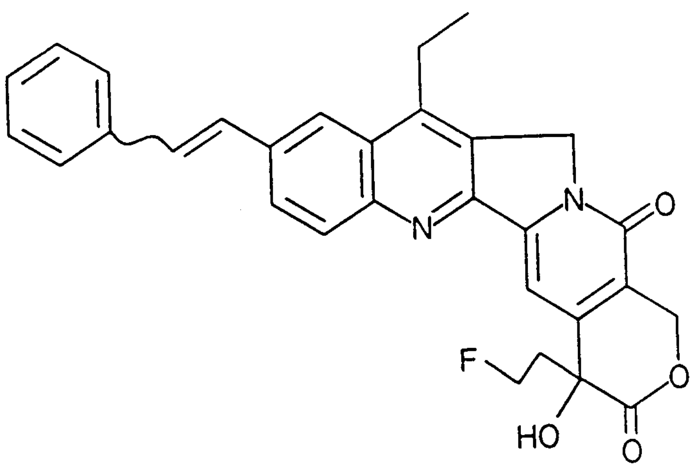

EXAMPLE 15

Synthesis of 9-cyano-11-ethyl-4-(2-fluoroethyl)-4-hydroxy-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.6 mg of 2-amino-5-cyanopropiophenone, a catalytic amount of p-toluenesulfonic acid, and 0.5 ml of toluene were added. The mixture was heated and refluxed for 2 hours. The reaction mixture was concentrated, and the concentration residue was purified with a silica gel column (eluting solvent: chloroform to chloroform-methanol (10:1)) to give 2.5 mg of the intended product in a pale brown crystal state.
H-NMR (in $CDCl_3$, δ, ppm)
1.44 (3H, t, $CH_2C\underline{H}_3$)
2.25~2.35 (2H, m, $C\underline{H}_2CH_2F$)
3.22 (2H, q, $C\underline{H}_2CH_3$)
4.54~4.84 (2H, m, $CH_2C\underline{H}_2F$)
5.27, 5.81 (2H, ABq, $C_1$-H)
5.30 (2H, s, $C_{12}$-H)
7.65 (1H, s, $C_5$-H)
7.95 (1H, dd, $C_8$-H)
8.32 (1H, d, $C_7$-H)
8.52 (1H, d, $C_{10}$-H)

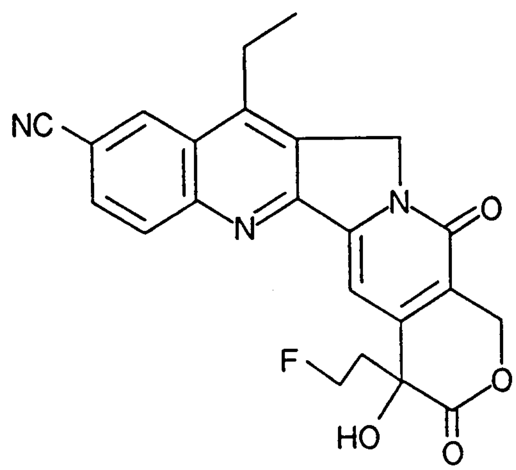

EXAMPLE 16

Synthesis of 11-ethyl-4-(2-fluoroethyl)-4-hydroxy-9-nitro-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid solution. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.9 mg of 2-amino-5-nitropropiophenone, a catalytic amount of p-toluenesulfonic acid, and 0.5 ml of toluene were added. The mixture was heated and refluxed for 2 hours. The reaction mixture was concentrated, and the concentration residue was purified with a silica gel column (eluting solvent: chloroform to chloroform-methanol (50:1)) to give 3.1 mg of the intended product.
H-NMR (in $CDCl_3$, δ, ppm)
1.47 (3H, t, $CH_2C\underline{H}_3$)

2.21~2.35 (2H, m, $C\underline{H}_2CH_2F$)
3.30 (2H, q, $C\underline{H}_2CH_3$)
4.63~4.81 (2H, m, $CH_2C\underline{H}_2F$)
5.33 (2H, s, $C_{12}$-H)
5.35, 5.81 (2H, ABq, $C_1$-H)
7.73 (1H, s, $C_5$-H)
8.37 (1H, d, $C_7$-H)
8.57 (1H, dd, $C_8$-H)
9.08 (1H, d, $C_{10}$-H)

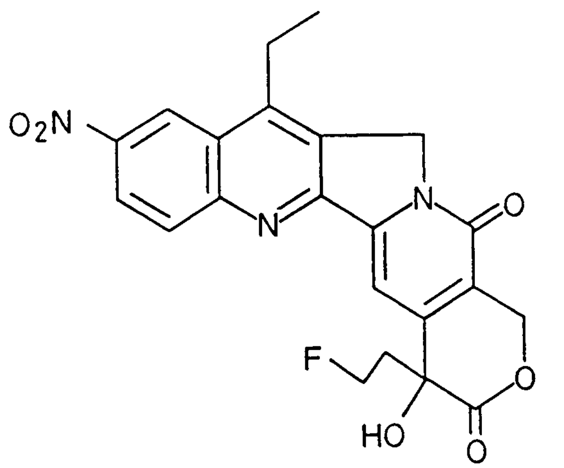

EXAMPLE 17

Synthesis of 9-amino-11-ethyl-4-(2-fluoroethyl)-4-hydroxy-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.5 mg of 2,5-diaminopropiophenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 1.1 mg of the intended product in a brown crystal state.
MS m/z=409 ($M^+$)
H-NMR (in $CDCl_3$, δ, ppm)
1.37 (3H, t, $CH_2C\underline{H}_3$)
2.21~2.38 (2H, m, $C\underline{H}_2CH_2F$)
3.09 (2H, q, $C\underline{H}_2CH_3$)
4.55~4.84 (2H, m, $CH_2C\underline{H}_2F$)
5.20 (2H, s, $C_{12}$-H)
5.33, 5.79 (2H, ABq, $C_1$-H)
7.14 (1H, d, $C_{10}$-H)
7.24 (1H, dd, $C_8$-H)
7.57 (1H, s, $C_5$-H)
8.03 (1H, d, $C_7$-H)

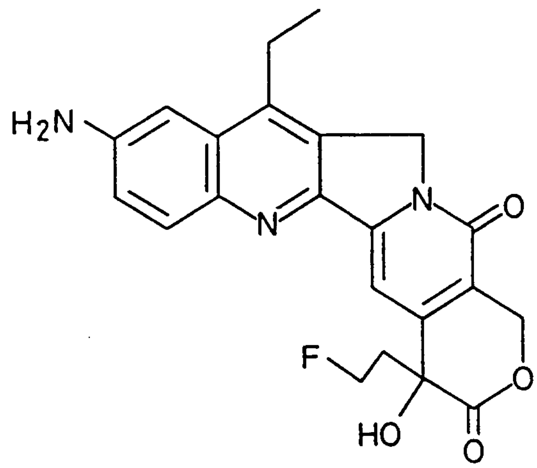

EXAMPLE 18

Synthesis of 11-ethyl-4-(2-fluoroethyl)-4-hydroxy-9-(N,N-dimethylamino)-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.6 mg of 2-amino-5-(N,N-dimethylamino)propiophenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 2.5 mg of the intended product in a yellowish brown crystal state.

MS m/z=437 ($M^+$)
H-NMR (in $CDCl_3$, δ, ppm)
1.38 (3H, t, $CH_2C\underline{H_3}$)
2.20~2.32 (2H, m, $C\underline{H_2}CH_2F$)
3.11 (2H, q, $C\underline{H_2}CH_3$)
3.17 (6H, s, $N(C\underline{H_3})_2$)
4.54~4.84 (2H, m, $CH_2C\underline{H_2}F$)
5.19 (2H, s, $C_{12}$-H)
5.33, 5.78 (2H, ABq, $C_1$-H)
7.14 (1H, d, $C_{10}$-H)
7.24 (1H, dd, $C_8$-H)
7.57 (1H, s, $C_5$-H)
8.03 (1H, d, $C_7$-H)

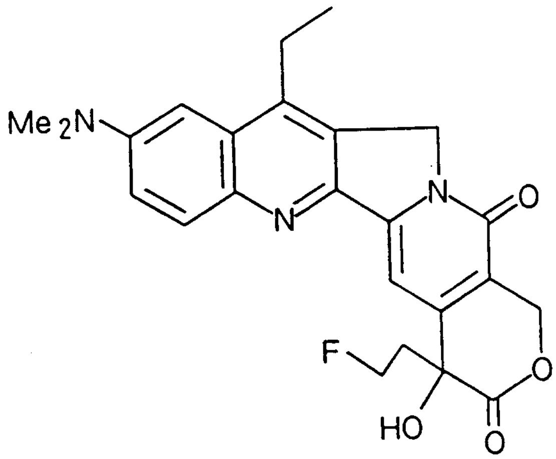

EXAMPLE 19

Synthesis of 11-ethyl-4-(2-fluoroethyl)-4-hydroxy-9-(4-methyl-1-piperazinyl)-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.8 mg of 2-amino-5-(4-methyl-1-piperazinyl)propiophenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 1,1 mg of the intended product in a yellowish brown crystal state.

MS m/z=492 ($M^+$)
H-NMR (in $CDCl_3$, δ, ppm)
1.18 (3H, t, $CH_2C\underline{H}_3$)
2.20~2.36 (2H, m, $C\underline{H}_2CH_2F$)
2.43 (3H, s, >$NC\underline{H}_3$)
2.73 (4H, m, piperazine-H)
3.06 (2H, q, $C\underline{H}_2CH_3$)
3.42 (4H, m, piperazine-H)
4.55~4.85 (2H, m, $CH_2C\underline{H}_2F$)
5.16 (2H, s, $C_{12}$-H)
5.27, 5.73 (2H, ABq, $C_1$-H)
7.22 (1H, d, $C_{10}$-H)
7.56 (1H, dd, $C_8$-H)
7.58 (1H, s, $C_5$-H)
8.08 (1H, d, $C_{12}$-H)

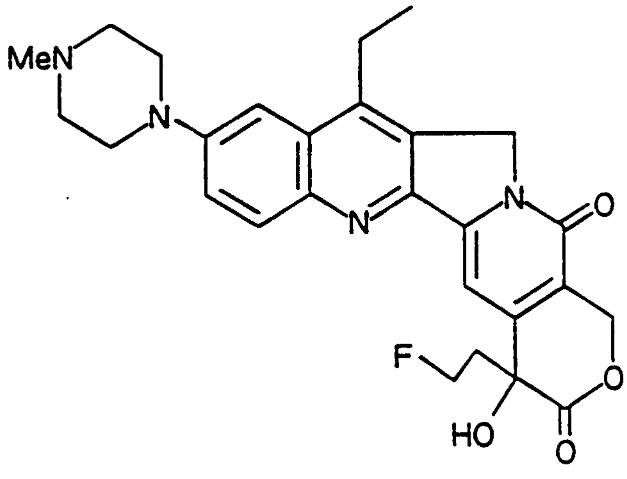

EXAMPLE 20

Synthesis of 11-ethyl-4-(2-fluoroethyl)-4-hydroxy-9-{(3-(N,N-dimethylaminomethyl)-1-pyrrolidinyl}-1H-pyrano-[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.1 mg of 2-amino-5-{3-(N,N-dimethylaminomethyl)-1-pyrrolidinyl}propiophenone and 0.2 ml of acetic acid were added. The mixture was stirred at 90°C for 3 hours. The reaction mixture was concentrated. To the concentration residue, methylene chloride and water were added. After neutralization with concentrated aqueous ammonia, the methylene chloride layer was separated. The methylene chloride layer was washed with water and was concentrated. The concentration residue was purified by preparative TLC (developing solvent: chloroform-methanol (10:1)) to give 3.2 mg of the intended product in a yellowish red crystal state.

H-NMR (in $CDCl_3$. δ. ppm)
1.36 (3H, t, $CH_2C\underline{H}_3$)
1.81~1.89, 2.18~2.66

(5H. m,
$C\underline{H}_2CH_2F$ 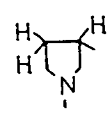 )

2.32 (6H, s, $N(C\underline{H}_3)_2$)
3.08 (2H, q, $C\underline{H}_2CH_3$)
3.21~3.25, 3.49~3.70

(6 H, m, 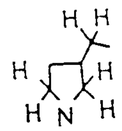 )

4.57~4.85 (2H, m, $CH_2C\underline{H_2}F$)
5.18 (2H, s, $C_{12}$-H)
5.32, 5.78 (2H, ABq, $C_1$-H)
6.76 (1H, d, $C_{10}$-H)
7.27 (1H, dd, $C_8$-H)
7.53 (1H, s, $C_5$-H)
8.04 (1H, d, $C_7$-H)

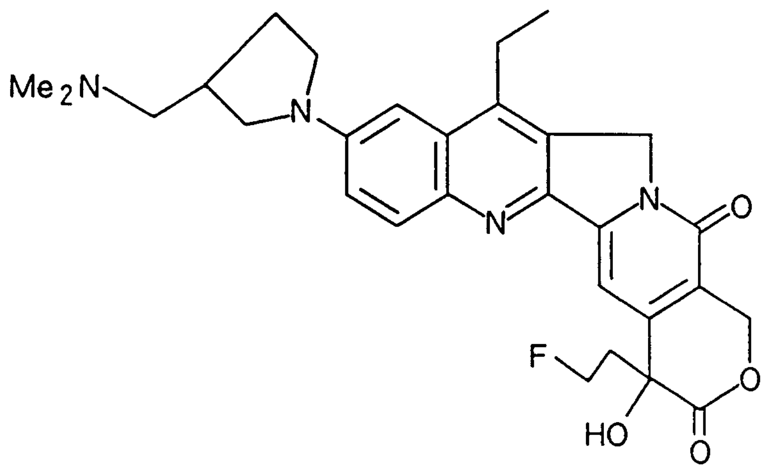

EXAMPLE 21

Synthesis of 3-benzyl-9-(2-fluoroethyl)-1,2-dihydro-9-hydroxy-4-methoxy-3H,12H-pyrano[3',4':6,7]indolizino[1,2-c]benzo[i,j][2,7]naphthyridine-10,13(9H,15H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.2 mg of 5-amino-1-benzyl-1,2,3,4-tetrahydro-8-methoxy-4-quinolinone, a catalytic amount of p-toluenesulfonic acid, and 0.5 ml of toluene were added. The mixture was heated and refluxed for 1.5 hours. The reaction mixture was concentrated, and the concentration residue was purified by a silica gel column (eluting solvent: chloroform to chloroform-methanol (25:1) to (10:1)) to give 1.8 mg of the intended product in a yellow crystal state.
H-NMR (in $CDCl_3$. δ. ppm)
2.21~2.32 (2H, m, $C\underline{H_2}CH_2F$)
3.00, 3.41 (2Hx2, tx2, $C_1$, $C_2$-H)
4.08 (3H, s, $OC\underline{H_3}$)
4.43 (2H, s, $C\underline{H_2}Ph$)
4.59~4.72 (2H, m, $CH_2C\underline{H_2}F$)
5.15 (2H, s, $C_{15}$-H)

5.34, 5.79 (2H, ABq, $C_{12}$-H)
7.29~7.38

(3 H, m, 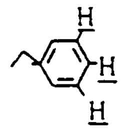 )

7.48

(2 H, d, 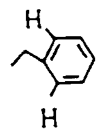 )

7.62 (1H, d, $C_5$-H)
7.63 (1H, s, $C_8$-H)
7.91 (1H, d, $C_6$-H)

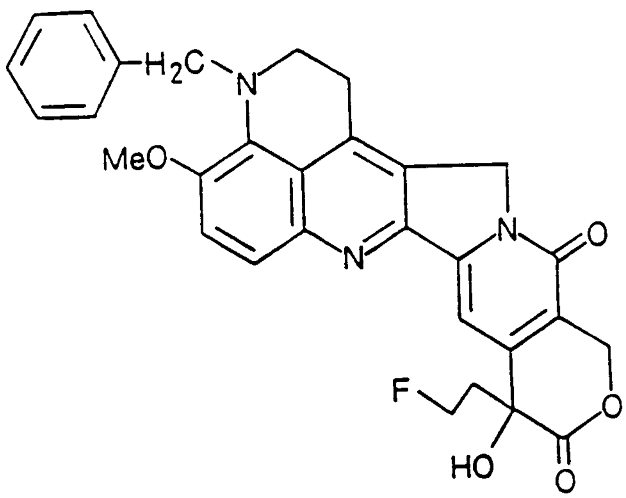

EXAMPLE 22

Synthesis of 9-(2-fluoroethyl)-1,2-dihydro-9-hydroxy-4-methoxy-12H-thiino[4,3,2-d,e]pyrano[3',4':6,7]indolizino[1,2-b]quinoline-10,13(9H,15H)-dione

4.3 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.6 mg of 5-amino-8-methoxy-4-thiochromanone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 0.8 mg of the intended product in a yellow crystal state.

H-NMR (in $CDCl_3$, δ, ppm)
2.20~2.34 (2H, m, C<u>H</u>$_2$CH$_2$F)
3.21, 3.45 (2H×2, t×2, $C_1C_2$-H)
4.05 (3H, s, OC<u>H</u>$_3$)
4.50~4.80 (2H, m, CH$_2$C<u>H</u>$_2$F)
5.17 (2H, s, $C_{15}$-H)
5.32, 5.74 (2H, ABq, $C_{12}$-H)
7.51 (1H, d, $C_5$-H)
7.63 (1H, s, $C_8$-H)
7.98 (1H, d, $C_6$-H)

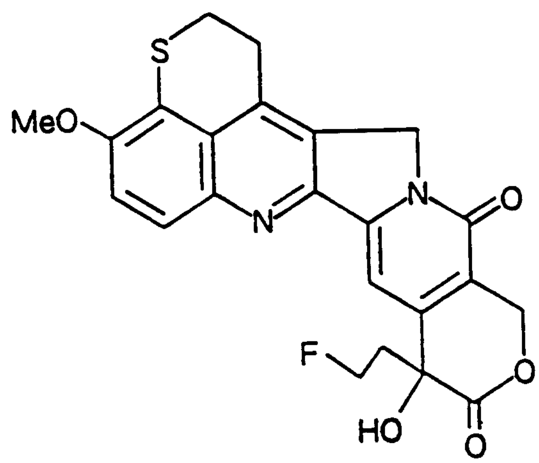

EXAMPLE 23

Synthesis of 9-(2-fluoroethyl)-1,2-dihydro-9-hydroxy-4-methoxy-12H-pyrano[4,3,2-d,e]pyrano[3',4':6,7]indolizino[1,2-b]quinoline-10,13(9H,15H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.3 mg of 5-amino-8-methoxy-4-chromanone, a catalytic amount of p-toluenesulfonic acid, and 0.7 ml of toluene were added. The mixture was heated and refluxed for 1 hour. The reaction mixture was concentrated, and the concentration residue was purified by preparative TLC (developing solvent: chloroform-methanol (25:1)) to give 1.6 mg of the intended product in a yellow crystal state.
H-NMR (in $CDCl_3$, δ, ppm)
2.18~2.35 (2H, m, C<u>H</u>$_2$CF$_2$F)
3.32 (2H, t, $C_1$-H)
4.07 (3H, s, $OCH_3$)
4.59 (2H, t, $C_2$-H)
4.59~4.81 (2H, m, CH$_2$C<u>H</u>$_2$F)
5.19 (2H, s, $C_{15}$-H)
5.34, 5.79 (2H, ABq, $C_{12}$-H)
7.60 (1H, d, $C_5$-H)
7.64 (1H, s, $C_8$-H)
7.85 (1H, d, $C_6$-H)

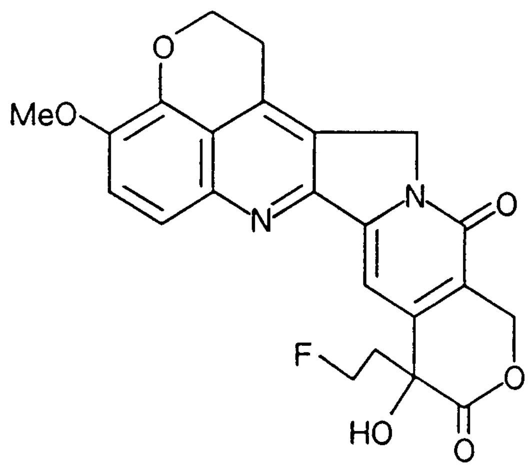

EXAMPLE 24

Synthesis of 4-chloro-9-(2-fluoroethyl)-2,3-dihydro-9-hydroxy-1H,12H-benzo[d,e]pyrano[3',4':6,7]indolizino[1,2-b]quinoline-10,13(9H,15H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.0 mg of 8-amino-5-chloro-1,2,3,4-tetrahydro-1-naphthalenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 3.2 mg of the intended product in a red crystal state.

H-NMR (in $CDCl_3$, δ, ppm)
2.19~2.41 (4H, m, $C\underline{H}_2CH_2F$, $C_2$-H)
3.13, 3.22 (2H×2, t×2, $C_1$, $C_3$-H)
4.56~4.79 (2H, m, $CH_2C\underline{H}_2F$)
5.20 (2H, s, $C_{15}$-H)
5.32, 5.75 (2H, ABq, $C_{12}$-H)
7.66 (1H, s, $C_8$-H)
7.73 (1H, d, $C_5$-H)
7.97 (1H, d, $C_6$-H)

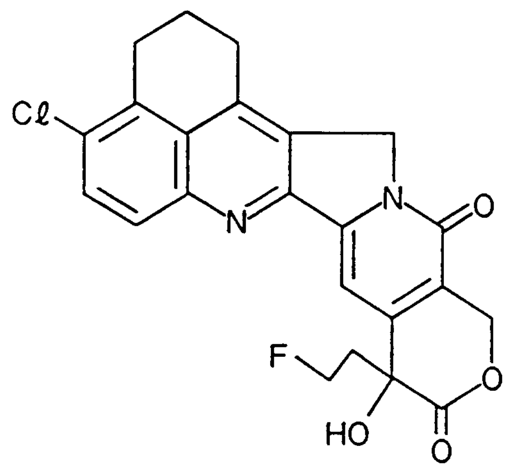

EXAMPLE 25

Synthesis of 9,11-diethyl-4-(2-fluoroethyl)-4-hydroxy-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.9 mg of 2-amino-5-ethylpropiophenone, a catalytic amount of p-toluenesulfonic acid, and 0.7 ml of toluene were added. The mixture was heated and refluxed for 2 hours. The reaction mixture was concentrated, and the concentration residue was purified by preparative TLC (developing solvent: chloroform-methanol (25:1)) to give 3.8 mg of the intended product in a pale brown crystal state.

H-NMR (in $CDCl_3$, δ, pm)
1.37~1.43 (6H, m, $CH_2C\underline{H_3}$x2)
2.18~2.33 (2H, m, $C\underline{H_2}CH_2F$)
2.92 (2H, q, $C_9$-$C\underline{H_2}CH_3$)
3.20 (2H, q, $C_{11}$-$C\underline{H_2}CH_3$)
4.58~4.82 (2H, m, $CH_2C\underline{H_2}F$)
5.25 (2H, s, $C_{12}$-H)
5.34, 5.80 (2H, ABq, $C_1$-H)
7.66 (1H, s, $C_5$-H)
7.68 (1H, dd, $C_8$-H)
7.87 (1H, d, $C_{10}$-H)
8.15 (1H, d, $C_7$-H)

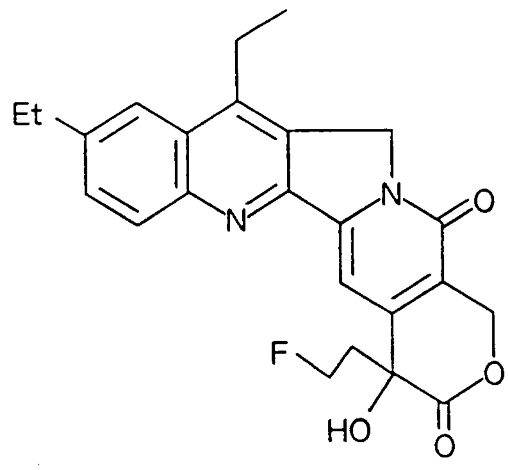

EXAMPLE 26

Synthesis of 9-ethoxy-11-ethyl-4-[2-fluoroethyl)-4-hydroxy-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.3 mg of 2-amino-5-ethoxypropiophenone, a catalytic amount of p-toluenesulfonic acid, and 0.7 ml of toluene were added. The mixture was heated and refluxed for 2 hours. The reaction mixture was concentrated, and the concentration residue was purified by preparative TLC (developing solvent: chloroform-methanol (25:1)) to give 3.6 mg of the intended product in a pale brown crystal state.

H-NMR (in $CDCl_3$, δ, ppm)
1.39 (3H, t, $CH_2C\underline{H}_3$)
1.54 (3H, t, $OCH_2C\underline{H}_3$)
2.21~2.32 (2H, m, $C\underline{H}_2CH_2F$)
3.13 (2H, q, $C\underline{H}_2CH_3$)
4.22 (2H, q, $OC\underline{H}_2CH_3$)
4.57~4.81 (2H, m, $CH_2C\underline{H}_2F$)
5.23 (2H, s, $C_{12}$-H)
5.33, 5.79 (2H, ABq, $C_1$-H)
7.30 (1H, d, $C_{10}$-H)
7.46 (1H, dd, $C_8$-H)
7.60 (1H, s, $C_5$-H)
8.12 (1H, d, $C_7$-H)

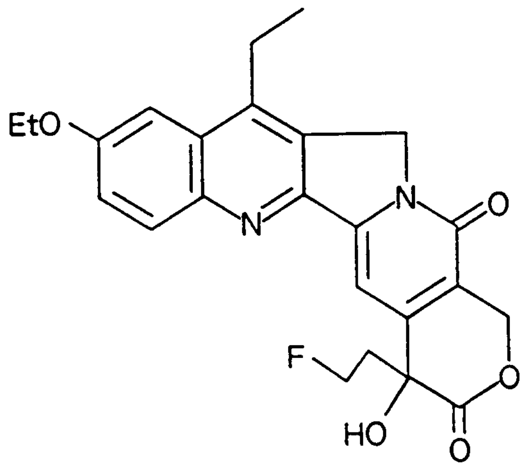

EXAMPLE 27

Synthesis of 11-ethyl-4-(2-fluoroethyl)-4-hydroxy-8-methyl-1H-pyrano[3',4':6,7]indolizino[1,2-b]quinoline-3,14(4H,12H)-dione

6.1 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.1 mg of 2-amino-4-methylpropiophenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (25:1)) to give 2.9 mg of the intended product in a pale brown crystal state.

H-NMR (in $CDCl_3$, δ, ppm)
1.40 (3H, t, $CH_2C\underline{H_3}$)
2.23~2.30 (2H, m, $C\underline{H_2}CH_2F$)
2.61 (3H, s, $C\underline{H_3}$)
3.18 (2H, q, $C\underline{H_2}CH_3$)
4.59~4.81 (2H, m, $CH_2C\underline{H_2}F$)
5.24 (2H, s, $C_{12}$-H)
5.34, 5.80 (2H, ABq, $C_1$-H)
7.51 (1H, dd, $C_9$-H)
7.65 (1H, S, $C_5$-H)
8.00~8.03 (2H, m, $C_7$, $C_{10}$-H)

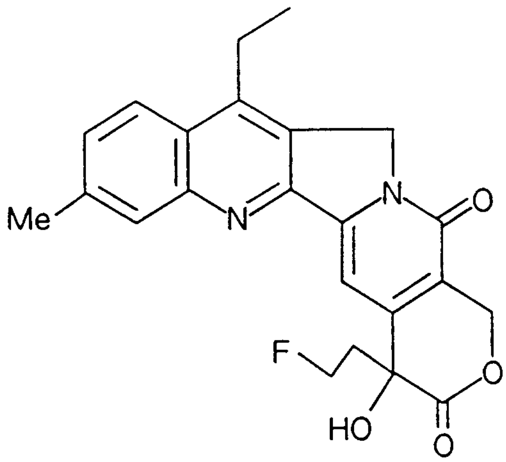

EXAMPLE 28

Synthesis of 11-ethyl-4-(2-fluoroethyl)-4-hydroxy-8-methoxy-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.5 mg of 2-amino-4-methoxypropiophenone, a catalytic amount of p-toluenesulfonic acid, and 0.5 ml of toluene were added. The mixture was heated and refluxed for 3 hours. The reaction mixture was concentrated, and the concentration residue was purified by preparative TLC (developing solvent: chloroform-methanol (25:1)) to give 4.4 mg of the intended product in a pale brown crystal state.

H-NMR (in $CDCl_3$, δ, ppm)
1.39 (3H, t, $CH_2C\underline{H_3}$)
2.21~2.32 (2H, m, $C\underline{H_2}CH_2F$)
3.17 (2H, q, $C\underline{H_2}CH_3$)
4.01 (3H, s, $OCH_3$)
4.58~4.83 (2H, m, $CH_2C\underline{H_2}F$)
5.23 (2H, s, $C_{12}$-H)
5.34, 5.80 (2H, ABq, $C_1$-H)
7.33 (1H, dd, $C_9$-H)
7.54 (1H, d, $C_7$-H)
7.65 (1H, s, $C_5$-H)
8.01 (1H, d, $C_{10}$-H)

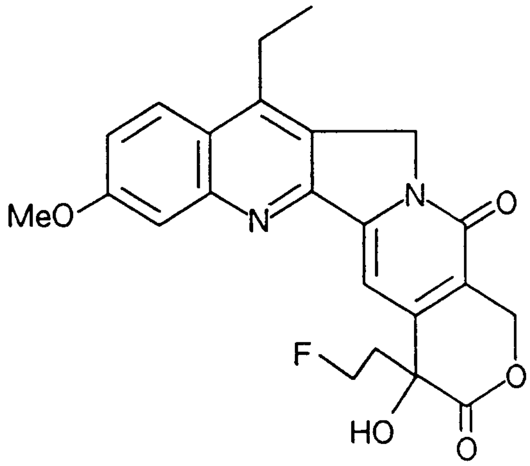

EXAMPLE 29

Synthesis of 11-ethyl-8-fluoro-4-(2-fluoroethyl)-4-hydroxy-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid, The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.7 mg of 2-amino-4-fluoropropiophenone, a catalytic amount of p-toluenesulfonic acid, and 0.7 ml of toluene were added. The mixture was heated and refluxed for 6 hours. The reaction mixture was concentrated, and the concentration residue was purified by preparative TLC (developing solvent: chloroform-methanol (25:1)) to give 3.7 mg of the intended product in a pale brown crystal state.

H-NMR (in $CDCl_3$, δ, ppm)
1.41 (3H, t, $CH_2C\underline{H_3}$)
2.21~2.33 (2H, m, $C\underline{H_2}CH_2F$)
3.20 (2H, q, $C\underline{H_2}CH_3$)
4.59~4.81 (2H, m, $CH_2C\underline{H_2}F$)
5.26 (2H, s, $C_{12}$-H)
5.34, 5.80 (2H, ABq, $C_1$-H)
7.47 (1H, d, d, d, $C_9$-H)
7.66 (1H, s, $C_5$-H)
7.84 (1H, dd, $C_7$-H)
8.13 (1H, dd, $C_{10}$-H)

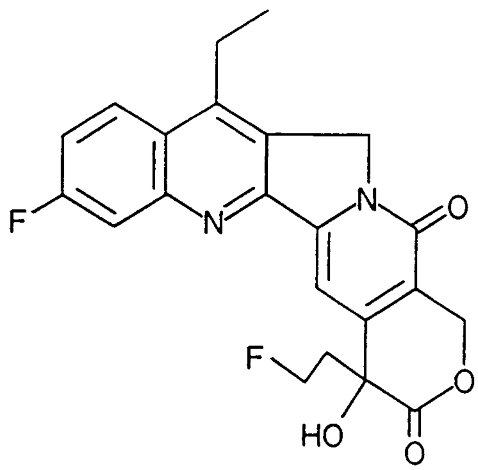

EXAMPLE 30

Synthesis of 8-chloro-11-ethyl-4-(2-fluoroethyl)-4-hydroxy-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid, The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.1 mg of 2-amino-4-chloropropiophenone, a catalytic amount of p-toluenesulfonic acid, and 0.7 ml of toluene were added. The mixture was heated and refluxed for 6 hours. The reaction mixture was concentrated, and the concentration residue was purified by preparative TLC (developing solvent: chloroform-methanol (25:1)) to give 3.7 mg of the intended product in a pale brown crystal state.

H-NMR (in $CDCl_3$, δ, ppm)

1.40 (3H, t, $CH_2C\underline{H_3}$)
2.21~2.33 (2H, m, $C\underline{H_2}CH_2F$)
3.1 9(2H, q, $C\underline{H_2}CH_3$)
4.59~4.81 (2H, m, $CH_2C\underline{H_2}F$)
5.25 (2H, s, $C_{12}$-H)
5.34, 5.80 (2H, ABq, $C_1$-H)
7.61 (1H, dd, $C_9$-H)
7.64 (1H, s, $C_5$-H)
8.05 (1H, d, $C_{10}$-H)
8.20 (1H, d, $C_7$-H)

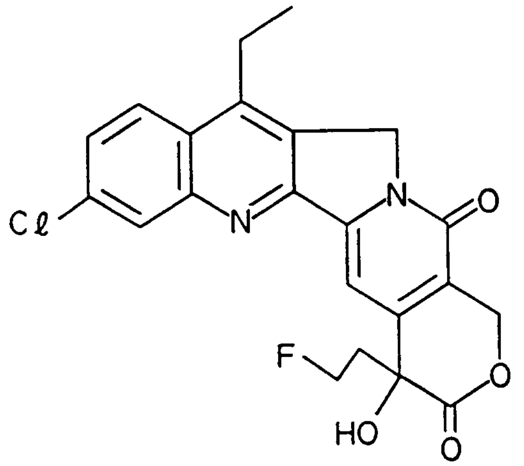

EXAMPLE 31

Synthesis of 8-bromo-11-ethyl-4-(2-fluoroethyl)-4-hydroxy-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 5.0 mg of 2-amino-4-bromopropiophenone, a catalytic amount of p-toluenesulfonic acid, and 0.7 ml of toluene were added. The mixture was heated and refluxed for 6 hours. The reaction mixture was concentrated, and the concentration residue was purified by preparative TLC (developing solvent: chloroform-methanol (25:1)) to give 4.3 mg of the intended product in a pale brown crystal state.

H-NMR (in $CDCl_3$, δ, ppm)
1.40 (3H, t, $CH_2C\underline{H_3}$)
2.21~2.33 (2H, m, $C\underline{H_2}CH_2F$)
3.19 (2H, q, $C\underline{H_2}CH_3$)
4.59~4.81 (2H, m, $CH_2C\underline{H_2}F$)
5.24 (2H, s, $C_{12}$-H)
5.34, 5.79 (2H, ABq, $C_1$-H)
7.64 (1H, s, $C_5$-H)
7.74 (1H, d, d, $C_9$-H)
7.98 (1H, d, $C_{10}$-H)
8.39 (1H, d, $C_7$-H)

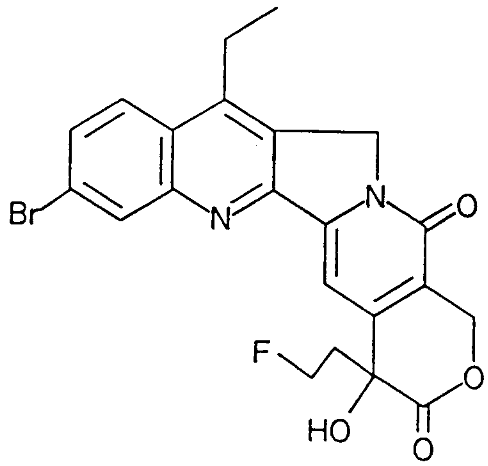

EXAMPLE 32

Synthesis of 11-ethyl-4-(2-fluoroethyl)-4-hydroxy-8-(N,N-dimethylamino)-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.7 mg of 2-amino-4-(N,N-dimethylamino)propiophenone, a catalytic amount of p-toluenesulfonic acid, and 0.5 ml of toluene were added. The mixture was heated and refluxed for 3 hours. The reaction mixture was concentrated, and the concentration residue was purified by preparative TLC (developing solvent: chloroform-methanol (25:1)) to give 3.4 mg of the intended product in an orange crystal state.

H-NMR (in $CDCl_3$, δ, ppm)
1.37 (3H, t, $CH_2\underline{CH_3}$)
2.22~2.31 (2H, m, $\underline{CH_2}CH_2F$)
3.12 (2H, q, $\underline{CH_2}CH_3$)
3.16 (6H, s, $N(CH_3)_2$)
4.56~4.79 (2H, m, $CH_2\underline{CH_2}F$)
5.18 (2H, s, $C_{12}$-H)
5.32, 5.79 (2H, ABq, $C_1$-H)
7.23 (1H, d, $C_7$-H)
7.27 (1H, dd, $C_9$-H)
7.62 (1H, s, $C_5$-H)
7.95 (1H, d, $C_{10}$-H)

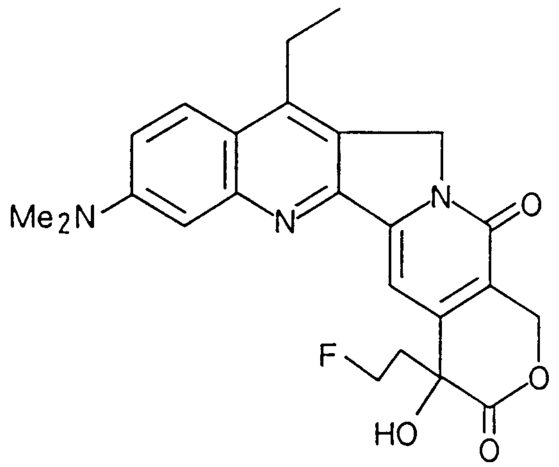

EXAMPLE 33

Synthesis of 11-ethyl-4-(2-fluoroethyl)-4-hydroxy-10-methoxy-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.9 mg of 2-amino-6-methoxy-propiophenone, a catalytic amount of p-toluenesulfonic acid, and 0.5 ml of toluene were added. The mixture was heated and refluxed for 5 hours. The reaction mixture was concentrated, and the concentration residue was purified by preparative TLC (developing solvent: chloroform-methanol (25:1)) to give 4.2 mg of the intended product in a pale brown crystal state.

H-NMR (in $CDCl_3$, δ, ppm)
1.36 (3H, t, $CH_2C\underline{H}_3$)
2.21~2.32 (2H, m, $C\underline{H}_2CH_2F$)
3.35~3.43 (2H, m, $C\underline{H}_2CH_3$)
4.03 (3H, s, $OCH_3$)
4.57~4.83 (2H, m, $CH_2C\underline{H}_2F$)
5.25 (2H, s, $C_{12}$-H)
5.34, 5.80 (2H, ABq, $C_1$-H)
6.90 (1H, dd, $C_9$-H)
7.63 (1H, S, $C_5$-H)
7.68 (1H, t, $C_8$-H)
7.80 (1H, dd, $C_7$-H)

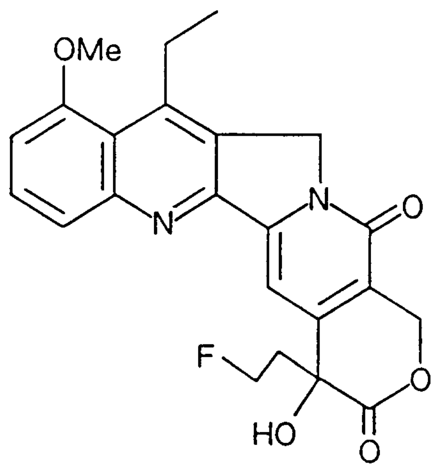

EXAMPLE 34

Synthesis of 11-ethyl-4-(2-fluoroethyl)-4-hydroxy-10-(N,N-dimethylamino)-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.2 mg of 2-amino-6-(N,N-dimethylamino)propiophenone, a catalytic amount of p-toluenesulfonic acid, and 0.7 ml of toluene were added. The mixture was heated and refluxed for 5 hours. The reaction mixture was concentrated, and the concentration residue was purified by preparative TLC (developing solvent: chloroform-methanol (25:1)) to give 4.4 mg of the intended product in a pale brown crystal state.

H-NMR (in $CDCl_3$, δ, ppm)
1.23 (3H, t, $CH_2C\underline{H}_3$)
2.21~2.33 (2H, m, $C\underline{H}_2CH_2F$)
2.78, 2.79 (3H×2, s×2, $N(C\underline{H}_3)_2$)
3.52~3.60 (2H, m, $C\underline{H}_2CH_3$)
4.58~4.82 (2H, m, $CH_2C\underline{H}_2F$)
5.30 (2H, s, $C_{12}$-H)
5.34, 5.81 (2H, ABq, $C_1$-H)
7.41 (1H, dd, $C_9$-H)
7.64 (1H, s, $C_5$-H)
7.69 (1H, dd, $C_8$-H)
7.95 (1H, dd, $C_7$-H)

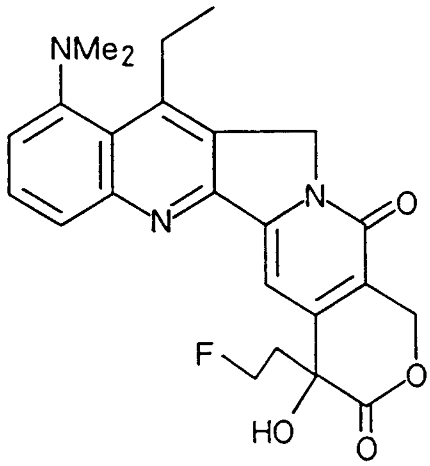

EXAMPLE 35

Synthesis of 4-(2-fluoroethyl)-4-hydroxy-9-methoxy-11-methyl-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.8 mg of 2-amino-5-methoxyacetophenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 3.8 mg of the intended product in a pale brown crystal state.

H-NMR (in $CDCl_3$, δ, ppm)
2.17~2.33 (2H, m, C$\underline{H}_2$$CH_2F$)
2.76 (3H, s, C$\underline{H}_3$)
4.01 (3H, s, OC$\underline{H}_3$)
4.58~4.81 (2H, m, $CH_2$C$\underline{H}_2$F)
5.21 (2H, s, $C_{12}$-H)
5.35, 5.81 (2H, ABq, $C_1$-H)
7.28 (1H, d, $C_{10}$-H)
7.48 (1H, dd, $C_8$-H)
7.61 (1H, s, $C_5$-H)
8.13 (1H, d, $C_7$-H)

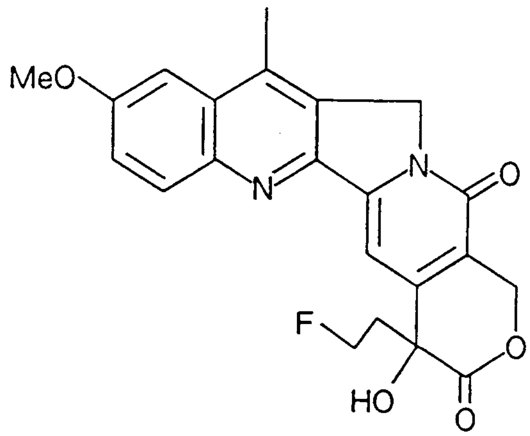

EXAMPLE 36

Synthesis of 4-(2-fluoroethyl)-4-hydroxy-9-methoxy-11-propyl-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.3 mg of 2-amino-5-methoxybutyrophenone, a catalytic amount of p-toluenesulfonic acid, and 0.7 ml of toluene were added. The mixture was heated and refluxed for 3 hours. The reaction mixture was concentrated, and the concentration residue was purified by preparative TLC (developing solvent: chloroform-methanol (25:1)) to give 4.2 mg of the intended product in a yellowish brown crystal state.

H-NMR (in $CDCl_3$, δ, ppm)
1.09 (3H, t, $CH_2CH_2C\underline{H}_3$)
1.81~1.87 (2H, m, $CH_2C\underline{H}_2CH_3$)
2.21~2.32 (2H, m, $C\underline{H}_2CH_2F$)
3.11 (2H, t, $C\underline{H}_2CH_2CH_3$)
4.00 (3H, s, $OC\underline{H}_3$)
4.57~4.81 (2H, m, $CH_2C\underline{H}_2F$)
5.24 (2H, s, $C_{12}$-H)
5.33, 5.80 (2H, ABq, $C_1$-H)
7.30 (1H, d, $C_{10}$-H)
7.46 (1H, dd, $C_8$-H)
7.61 (1H, s, $C_5$-H)
8.13 (1H, d, $C_7$-H)

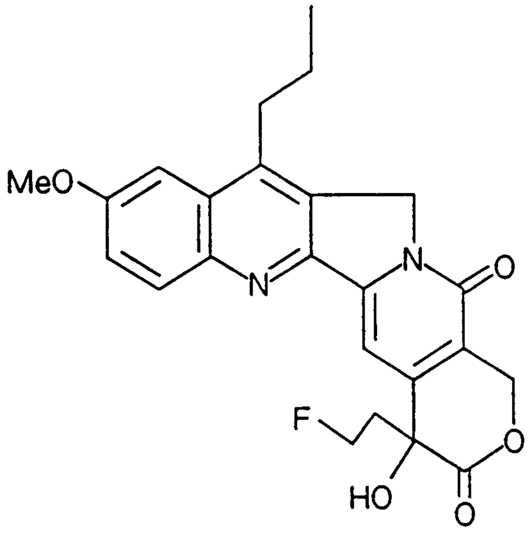

EXAMPLE 37

Synthesis of 4-(2-fluoroethyl)-4-hydroxy-1H-pyrano[3',4':6,7]indolizino[1,2-b]quinoline-3,14(4H,12H)-dione

31.5 mg of the compound obtained in Example 4 was dissolved in 1.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 11.8 mg of 2-aminobenzaldehyde and 1.5 ml of acetic acid were added. The mixture was stirred at 100°C for 3 hours. The reaction mixture was concentrated, and the concentration residue was suspended in acetonitrile, and collected by filtration, giving 13.9 mg of the intended product in a pale brown crystal state. The filtrate was purified by preparative TLC (developing solvent: chloroform-methanol (10:1)) to give further 6.5 mg of the intended product.

H-NMR (in $CDCl_3$, δ, ppm)
2.22~2.35 (2H, m, $C\underline{H}_2CH_2F$)
4.59~4.82 (2H, m, $CH_2C\underline{H}_2F$)
5.31 (2H, s, $C_{12}$-H)
5.35, 5.81 (2H, ABq, $C_1$-H)
7.68 (1H, ddd, $C_8$ or $C_9$-H)
7.70 (1H, s, $C_5$-H)
7.84 (1H, ddd, $C_8$ or $C_9$-H)
7.94 (1H, dd, $C_7$ or $C_{10}$-H)
8.24 (1H, dd, $C_7$ or $C_{10}$-H)
8.41 (1H, s, $C_{11}$-H)

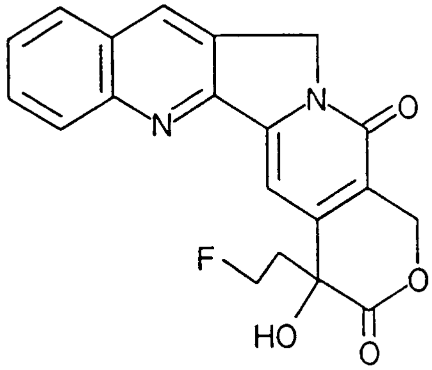

EXAMPLE 38

Synthesis of 4-[2-fluoroethyl)-4-hydroxy-11-hydroxymethyl-1H-pyrano[3',4':6,7]indolizino[1,2-b]quinoline-3,14[4H,12H)-dione

20.0 mg of the compound obtained in Example 37 was suspended in 0.6 ml of methanol, and thereto 0.5 ml of 75%-sulfuric acid and 0.5 ml of water were added to dissolve the compound. Thereto 15.3 mg of ferrous sulfate heptahydrate was added, and under ice cooling 0.1 ml of aqueous 35% hydrogen peroxide was gradually added. The resulting mixture was stirred at room temperature for 5 hours, and left standing overnight. Ice water was added to the reaction mixture. The precipitated crystalline matter was collected by filtration, washed with water, and vacuum-dried. Thus 13.3 mg of the intended product was obtained in a pale brown crystal state.

H-NMR (in DMSO-$d_6$, δ, ppm)
2.29 (2H, d, t, C$\underline{H}_2$C$H_2$F)
4.49~4.73 (2H, m, C$H_2$C$\underline{H}_2$F)
5.28, 5.43, 5.47 (2H×3, s×3, C$\underline{H}_2$OH, $C_1$-H, $C_{12}$-H)
7.36 (1H, s, $C_5$-H)
7.71, 7.86 (1H×2, ddd×2, $C_8$, $C_9$-H)
8.16~8.20 (2H, m, $C_7$, $C_{10}$-H)

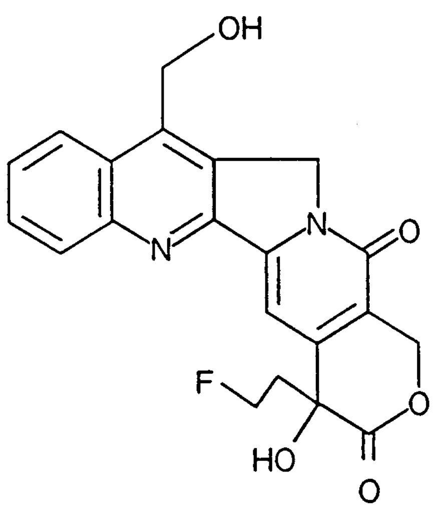

EXAMPLE 39

Synthesis of 4-(2-fluoroethyl)-11-formyl-4-hydroxy-1H-pyrano[3',4':6,7]indolizino[1,2-b]quinoline-3,14(4H,12H)-dione

8.3 mg of the compound obtained in Example 38 was suspended in 4 ml of acetic acid, and the mixture was heated and refluxed for 3 hours. The reaction mixture was concentrated, and the concentration residue was purified by preparative TLC (developing solvent: chloroform-methanol (10:1)). The obtained yellow crystalline matter was suspended in approximately 1 ml of ice water, and 0.6 ml of concentrated hydrochloric acid was added thereto. The mixture was stirred at room temperature for 10 hours, and further at 50°C for 1 hour. The reaction mixture was concentrated, and the concentration residue was purified by preparative TLC (developing solvent: chloroform-methanol (10:1)). Thus 3.1 mg of the intended product was obtained in a yellow crystal state.
H-NMR (in $CDCl_3$)
2.22~2.36 (2H, m, $C\underline{H}_2CH_2F$)
4.60~4.81 (2H, m, $CH_2C\underline{H}_2F$)
5.35, 5.82 (2H, ABq, $C_1$-H)
5.62 (2H, s, $C_{12}$-H)
7.70 (1H, s, $C_5$-H)
7.87, 7.93 (1Hx2, dddx2, $C_8$, $C_9$-H)
8.37 (1H, dd, $C_7$-H)
8.77 (1H, dd, $C_{10}$-H)
11.2 (1H, s, C<u>H</u>O)

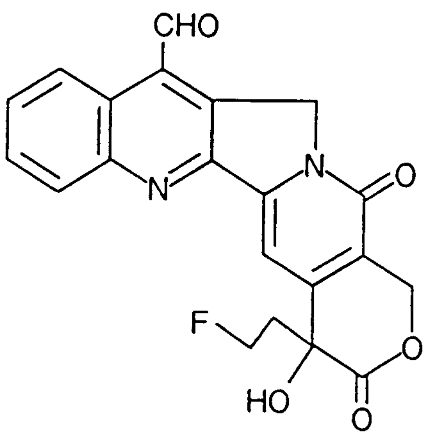

EXAMPLE 40

Synthesis of 11-acetoxymethyl-4-(2-fluoroethyl)-4-hydroxy-1H-pyrano[3',4':6,7]indolizino[1,2-b]quinoline-3,14(4H,12H)-dione

9.4 mg of the compound obtained in Example 38 was suspended in a mixture of 1 ml of pyridine and 1 ml of dimethylformamide, and 20 µl of acetic anhydride was added thereto. The resulting mixture was dissolved by heating, and stirred at room temperature for 1 hour. Further, 10 µl of acetic anhydride was added and the mixture was stirred for 1 hour. The reaction mixture was concentrated. The concentration residue was purified by preparative TLC (developing solvent: chloroform-methanol (10:1)). Thus 1.9 mg of the intended product was obtained in a pale brown crystal state.
H-NMR (in $CDCl_3$, δ, ppm)
2.19 (3H, s, $COC\underline{H}_3$)
2.22~2.35 (2H, m, $C\underline{H}_2CH_2F$)
4.59~4.83 (2H, m, $CH_2C\underline{H}_2F$)
5.34, 5.80 (2H, ABq, $C_1$-H)
5.47 (2H, s, $C_{12}$-H)
5.73 (2H, s, $C_{11}C\underline{H}_2OAc$)
7.54 (1H, s, $C_5$-H)

7.72, 7.85 (1H×2, ddd×2, $C_8$, $C_9$-H)
8.14, 8.26 (1H×2, d, $C_7$, $C_{10}$-H)

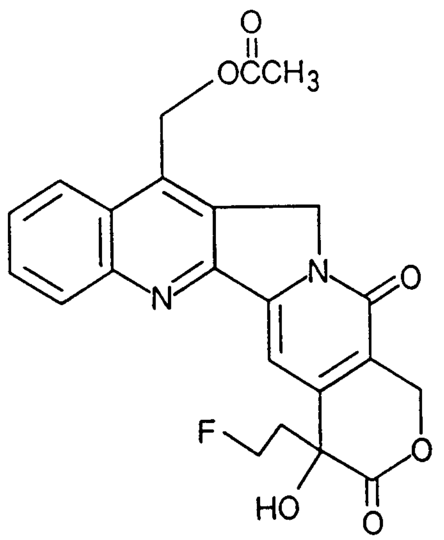

EXAMPLE 41

Synthesis of 4-[2-fluoroethyl)-4-hydroxy-11-methyl-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 1.9 mg of 2-aminoacetophenone, a catalytic amount of p-toluenesulfonic acid, and 0.5 ml of toluene were added. The mixture was heated and refluxed for 2 hours. The reaction mixture was concentrated, and the concentration residue was purified by preparative TLC (developing solvent: chloroform-methanol (25:1)) to give 4.6 mg of the intended product in a pale brown crystal state.
H-NMR (in $CDCl_3$, δ, ppm)
2.22~2.35 (2H, m, $C\underline{H}_2CH_2F$)
2.83 (3H, s, $C_{11}$-$C\underline{H}_3$)
4.59~4.82 (2H, m, $CH_2C\underline{H}_2F$)
5.28 (2H, s, $C_{12}$-H)
5.35, 5.81 (2H, ABq, $C_1$-H)
7.68 (1H, s, $C_5$-H)
7.70, 7.83 (1H×2, ddd×2, $C_8$, $C_9$-H)
8.13, 8.23 (1H×2, dd×2, $C_7$, $C_{10}$-H)

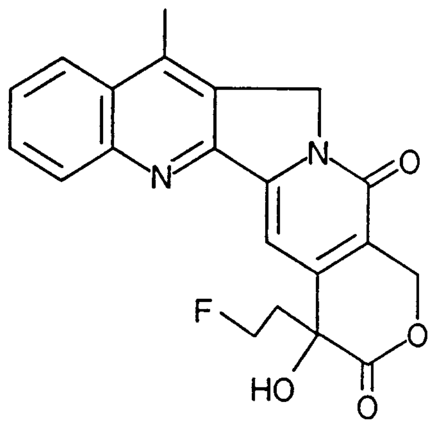

EXAMPLE 42

Synthesis of 4-(2-fluoroethyl)-4-hydroxy-11-propyl-1H-pyrano[3',4':6,7]indolizino[1,2-b]quinoline-3,14(4H,12H)-dione

5.0 mg of the compound obtained in Example 4 was dissolved in 0.5 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 2.6 mg of 2-aminobutyrophenone, a catalytic amount of p-toluenesulfonic acid, and 0.5 ml of toluene were added. The mixture was heated and refluxed for 2 hours. The reaction mixture was concentrated, and the concentration residue was purified by preparative TLC (developing solvent: chloroform-methanol (25:1)) to give 3.5 mg of the intended product in a yellow crystal state.

H-NMR (in $CDCl_3$, δ, ppm)

1.10 (3H, t, $C_{11}$-$CH_2CH_2C\underline{H}_3$)

1.81~1.87 (2H, m, $C_{11}$-$CH_2C\underline{H}_2CH_3$)

2.22~2.35 (2H, m, $C\underline{H}_2CH_2F$)

3.17 (3H, t, $C_{11}C\underline{H}_2CH_2CH_3$)

4.58~4.82 (2H, m, $CH_2C\underline{H}_2F$)

5.28 (2H, s, $C_{12}$-H)

5.34 ,5.80 (2H ,ABq, $C_1$-H)

7.68, 7.82 (1H×2, ddd×2, $C_8$, $C_9$-H)

7.70 (1H, s, $C_5$-H)

8.13, 8.25 (1H×2, dd×2, $C_7$, $C_{10}$-H)

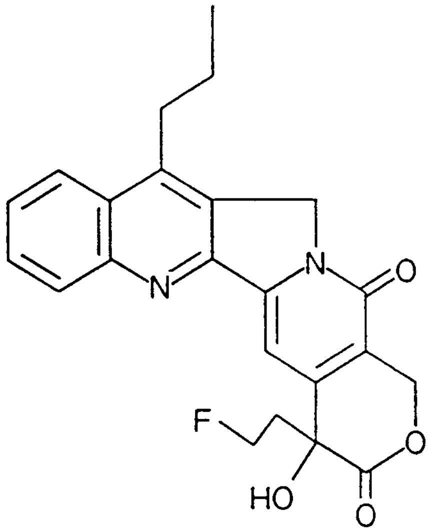

EXAMPLE 43

Synthesis of 11-ethyl-4-(2-fluoroethyl)-4,9-dihydroxy-1H-pyrano[3',4':6,7]indolizino[1,2-b]quinoline-3,14(4H,12H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.7 mg of 2-amino-5 hydroxypropiophenone and 0.7 ml of acetic acid were added. The mixture was stirred at 100°C for 2 hours. The reaction mixture was concentrated, and the concentration residue was purified by preparative TLC (developing solvent: chloroform-methanol (10:1)) to give 4.1 mg of the intended product in a pale brown crystal state.

H-NMR (in DMSO-$d_6$, δ, ppm)
1.30 (3H, t, $CH_2C\underline{H}_3$)
2.23~2.32 (2H, m, $C\underline{H}_2CH_2F$)
3.09 (2H, q, $C\underline{H}_2CH_3$)
4.48~4.72 (2H, m, $CH_2C\underline{H}_2F$)
5.29 (2H, s, $C_{12}$-H)
5.46 (2H, s, $C_1$-H)
6.80 (1H, s, O$\underline{H}$)
7.27 (1H, s, $C_5$-H)
7.41 (1H, d, $C_{10}$-H)
7.42 (1H, d, $C_8$-H)
8.03 (1H, d, $C_{12}$-H)
10.3 (1H, br, O$\underline{H}$)

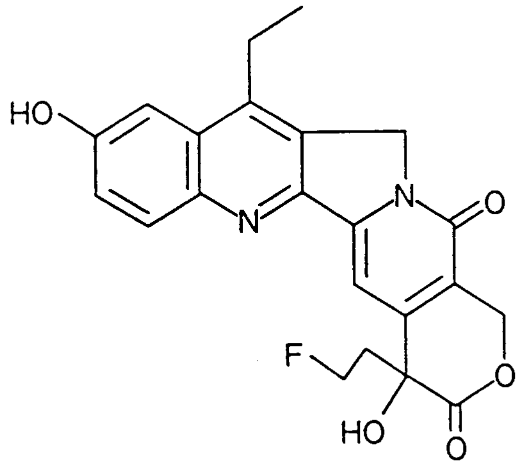

EXAMPLE 44

Synthesis of 9-(2-fluoroethyl)-2,3-dihydro-9-hydroxy-4-methoxy-1H,12H-benzo[d,e]pyrano[3',4':6,7]indolizino[1,2-b]quinoline-10,13(9H,15H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.1 mg of 8-amino-5-methoxy-1,2,3,4-tetrahydro-1-naphthalenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 7.4 mg of the intended product in a pale brown crystal state.

H-NMR (in DMSO-$d_6$. δ. ppm)
2.05~2.07 (2H, m, $C_2$-H)
2.31~2.39 (2H, m, $C\underline{H}_2CH_2F$)
3.03, 3.14 (2H×2, m×2, $C_1$, $C_3$-H)
4.01 (3H, s, $OC\underline{H}_3$)
4.52~4.79 (2H, m, $CH_2C\underline{H}_2F$)
5.23 (2H, s, $C_{15}$-H)
5.51 (2H, s, $C_{12}$-H)
6.86 (1H, s, OH)
7.33 (1H, s, $C_8$-H)
7.76 (1H, d, $C_5$-H)
8.06 (1H, d, $C_6$-H)

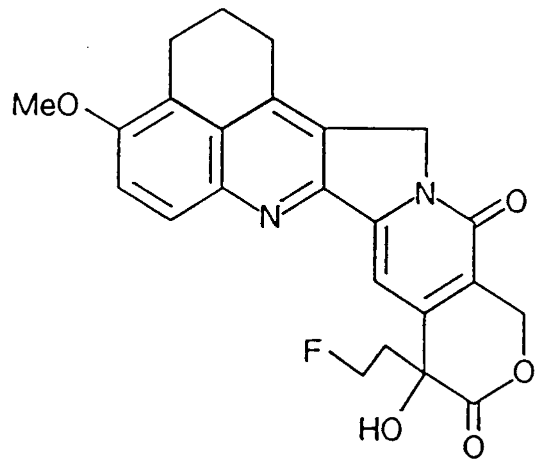

EXAMPLE 45

Synthesis of 4-ethoxy-9-(2-fluoroethyl)-2,3-dihydro-9-hydroxy-1H,12H-benzo[d,e]pyrano[3',4':6,7]indolizino[1,2-b]quinoline-10,13(9H,15H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 4.4 mg of 8-amino-5-ethoxy-1,2,3,4-tetrahydro-1-naphthalenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 5.0 mg of the intended product in a pale brown crystal state.

H-NMR (in DMSO-$d_6$. δ. ppm)
1.40 (3H, t, $OCH_2C\underline{H_3}$)
2.01~2.02 (2H, m, $C_2$-H)
2.26~2.32 (2H, m, $C\underline{H_2}CH_2F$)
2.99, 3.10 (2H×2, m×2, $C_1$, $C_3$-H)
4.14 (2H, q, $OC\underline{H_2}CH_3$)
4.50~4.73 (2H, m, $CH_2C\underline{H_2}F$)
5.19 (2H, s, $C_{15}$-H)
5.46 (2H, s, $C_{12}$-H)
6.81 (1H, s, OH)
7.29 (1H, s, $C_8$-H)
7.70 (1H, d, $C_5$-H)
7.98 (1H, d, $C_6$-H)

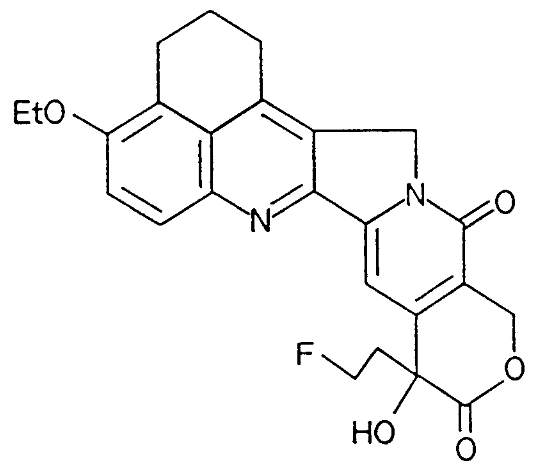

EXAMPLE 46

Synthesis of 9-(2-fluoroethyl)-2,3-dihydro-9-hydroxy-4-methyl-1H,12H-benzo[d,e]pyrano[3',4':6,7]indolizino[1,2-b]quinoline-10,13[9H,15H)-dione

7.0 mg of the compound obtained in Example 4 was dissolved in 0.7 ml of aqueous 80%-trifluoroacetic acid. The solution was stirred under an argon gas stream at room temperature for 1 hour. The reaction mixture was concentrated, and the resulting concentration residue was dissolved in methylene chloride. The solution was washed with water, and concentrated to dryness. To the resulting concentration residue, 3.8 mg of 8-amino-5-methyl-1,2,3,4-tetrahydro-1-naphthalenone, a catalytic amount of p-toluenesulfonic acid, and 0.5 ml of toluene were added. The mixture was heated and refluxed for 1 hour. The reaction mixture was concentrated, and the concentration residue was purified by preparative TLC (developing solvent: chloroform-methanol (10:1)) to give 5.4 mg of the intended product in a pale brown crystal state.

H-NMR (in DMSO-$d_6$. δ. ppm)
2.05~2.08 (2H, m, $C_2$-H)
2.26~2.32 (2H, m, $C\underline{H}_2CH_2F$)
2.42 (3H, s, $C\underline{H}_3$)
3.00, 3.12 (2H×2, m×2, $C_1$, $C_3$-H)
4.51~4.71 (2H, m, $CH_2C\underline{H}_2F$)
5.18 (2H, s, $C_{15}$-H)
5.46 (2H, s, $C_{12}$-H)
6.83 (1H, s, OH)
7.30 (1H, s, $C_8$-H)
7.64 (1H, d, $C_5$-H)
7.87 (1H, d, $C_6$-H)

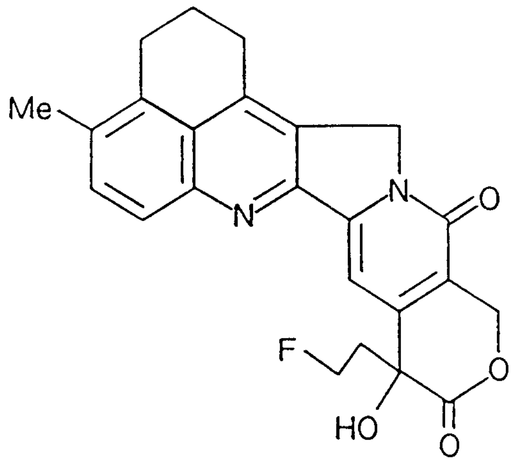

TEST EXAMPLE 1

Antineoplastic effect for P338 leukemia mice

P388 cells of $10^6$ in number were transplanted into abdominal cavity of $CDF_1$ mice of approximately 9 week-age (female, Non-dosed group: 5 - 8 mice, group dosed with the test compound: 1 - 5 mice). One, five, and nine days after the transplantation, the test compound which was dissolved or suspended in 1% Tween 80 (Kanto Chemical Inc.)/physiological saline was dosed to the abdominal cavity. The antineoplastic effect of the test compounds are shown by the T/C value (%) derived from the formula below.

$$\frac{T}{C}(\%) = \frac{\text{Average survival days of dosed group}}{\text{Average survival days of non-dosed group}} \times 100$$

Table 1

| Antineoplastic effect for P388 leukemia mice | | |
|---|---|---|
| Test compound | Total dose (mg/kg) | T/C (%) |
| Example 11 | 20 | 483 |
| Example 16 | 20 | 345 |
| Example 24 | 20 | 385 |
| Example 25 | 30 | 345 |
| Example 26 | 30 | 583 |
| Example 41 | 30 | 386 |
| Camptothecin | 20 | 218 |

As shown above, the compounds of the present invention exhibited higher antineoplastic effect.

## Claims

### Claims for the following Contracting States : BE, CH, DE, FR, GB, IT, LI, NL, SE

1. A compound represented by the general formula (I) below and a salt thereof:

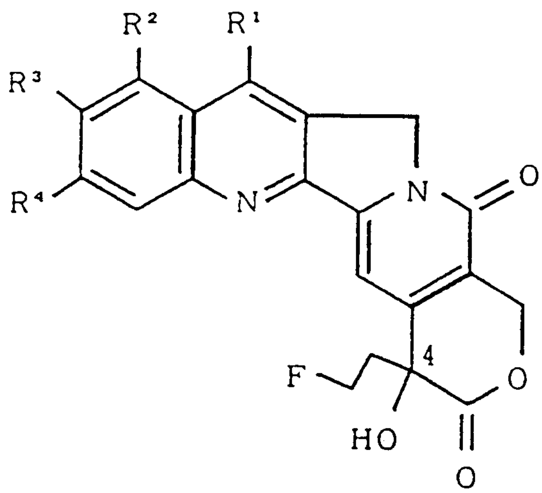

(I)

where $R^1$ is hydrogen, $C_1$-$C_6$ alkyl, hydroxymethyl, acyloxymethyl, and formyl; $R^2$, $R^3$, and $R^4$ are independently hydrogen, hydroxyl, $C_1$-$C_6$ alkyl, $C_2$-$C_6$ alkenyl, $C_2$-$C_6$ alkynyl, $C_1$-$C_6$ alkoxy, halogen, amino, $C_2$-$C_7$ acylamino, $C_1$-$C_6$ alkylamino, $NR^5R^6$ [ in which $R^5$ and $R^6$ are independently $C_1$-$C_6$ alkyl or may form a five-membered or six-membered ring together with a nitrogen atom bonded thereto or further together with an atom of O, S, or N-$R^7$ ($R^7$ being hydrogen, $C_1$-$C_6$ alkyl or an amino-protecting group] as a ring constituent, and a ring-constituting carbon may be substituted by $C_1$-$C_3$ alkyl, amino, or aminomethyl ], phenyl, naphthyl, or pyridyl, or $R^1$ and $R^2$ may form together a bridged structure represented by $-(CH_2)_m$-Z-$(CH_2)_n$- [ in which Z is O, S, CH-$R^8$ ($R^8$ being hydrogen or $C_1$-$C_6$ alkyl), or N-$R^{7'}$ ($R^{7'}$ being hydrogen, $C_1$-$C_6$ alkyl, or an amino-protecting group); and m and n are respectively 0, 1, or 2 provided that m and n are not simultaneously zero.].

2. The compound and the salt thereof of claim 1, wherein the steric configuration at the 4-position of the compound represented by the general formula (I) is the S-configuration.

3. A pyranoindolizine derivative represented by the general formula (II):

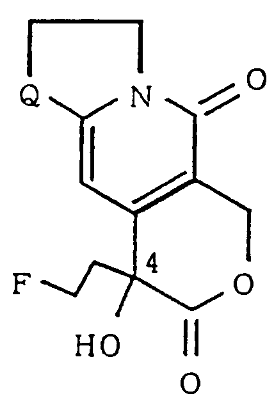

(II)

where Q is C=O or

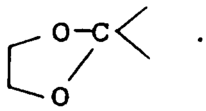

4. The compound of claim 3, wherein the steric configuration at the 4-position of the compound represented by the general formula (II) is the S-configuration.

5. A pyranoindolizine derivative represented by the general formula (III) below:

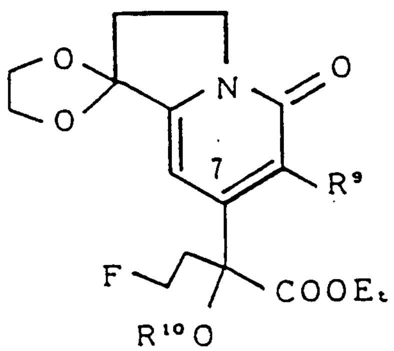

(III)

where $R^9$ is cyano, acetylaminomethyl, or acetoxymethyl; and $R^{10}$ is formyl, acetyl, benzoyl, or (R)-1-(p-toluenesulfonyl)prolyl.

6. The compound of claim 5, wherein the steric configuration at the α-position of the acetic acid group at the 7-position of the compound of the general formula (III) is the S-configuration.

7. A process for producing a pyranoindolizine derivative represented by the general formula ($II_1$):

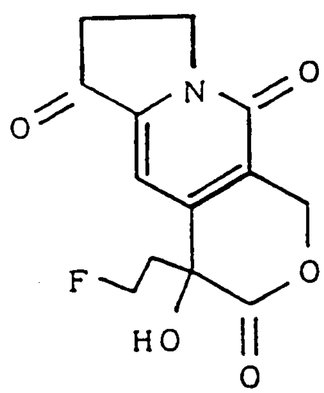

($II_1$)

comprising steps of 2-fluoroethylating the compound of the general formula (IV):

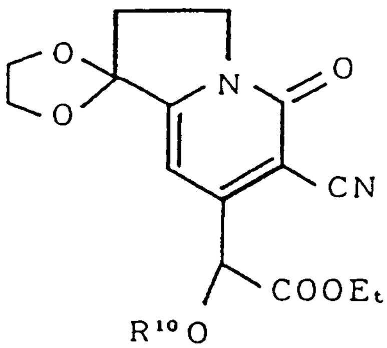

(IV)

where $R^{10}$ is formyl, acetyl, benzoyl, or (R)-1-(p-toluenesulfonyl)prolyl to produce the compound of the general formula ($III_3$):

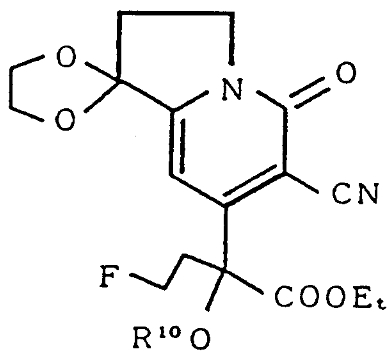

( $III_3$ )

reducing the compound of the general formula ($III_3$) to produce the compound of the general formula ($III_2$) below:

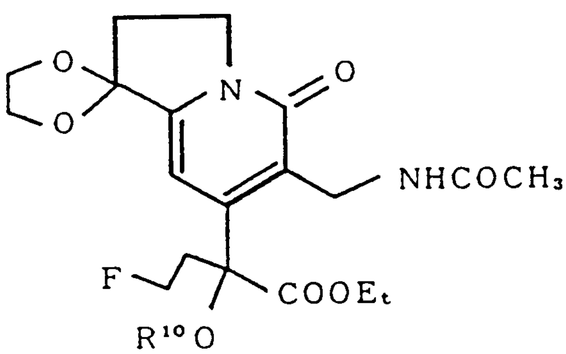

( $III_2$ )

rearranging the compound of the general formula ($III_2$) in the presence of a nitrosation agent to produce the compound of the general formula ($III_1$):

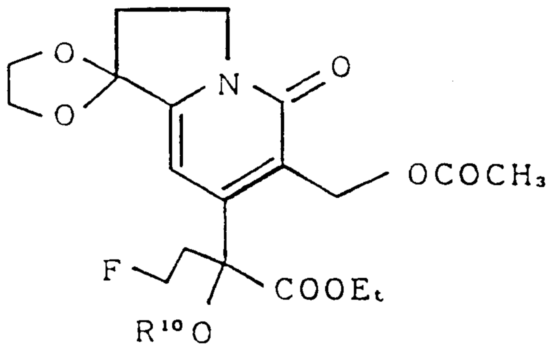

(III₁)

causing ring closure of the compound of the general formula ($III_1$) to produce the compound of the general formula ($II_2$)

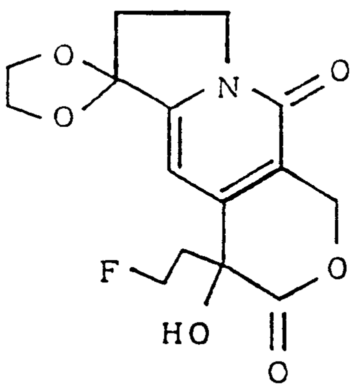

($II_2$)

and deketalizing the compound of the general formula ($II_2$). thereby producing the compound of the general formula ($II_1$).

8. The process for producing the compound of the general formula (I) according to claim 1 comprising the step of condensing the compound of the general formula ($II_1$):

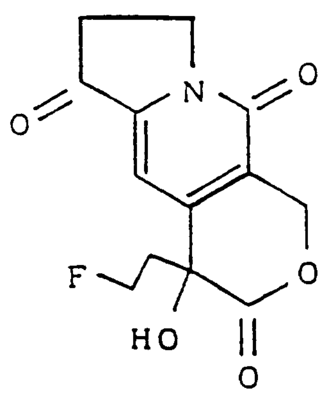

($II_1$)

with the compound of the general formula (V):

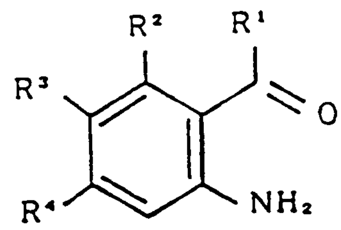

(V)

where $R^1$,$R^2$, $R^3$, and $R^4$ are defined as in claim 1.

**9.** Pharmaceutical compositions comprising a compound of any of claims 1 or 2 or their salts and pharmaceutically acceptable diluents and/or carriers.

**10.** A pharmaceutical composition according to claim 9 exhibiting antineoplastic activity.

**Claims for the following Contracting State : ES**

**1.** A process for producing the compound of the general formula (I):

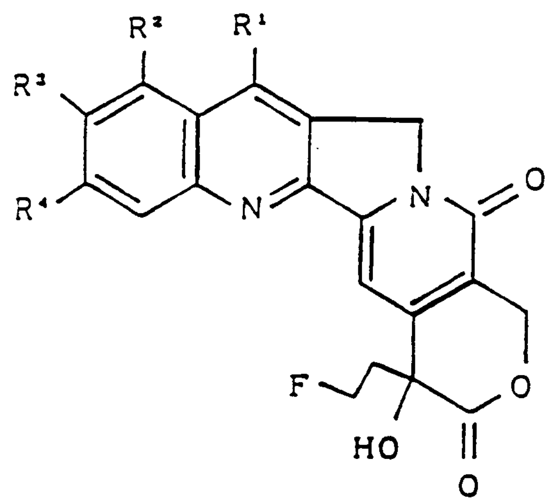

(I)

comprising the step of condensing the compound or the general formula ($II_1$):

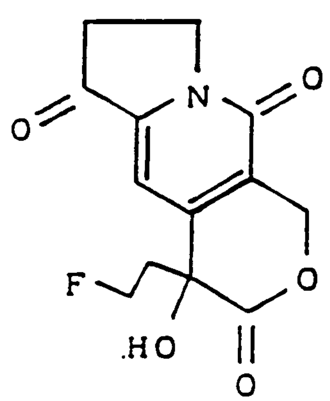

($II_1$)

with the compound of the general formula (V):

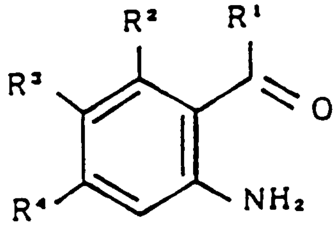

(V)

where $R^1$ is hydrogen, $C_1$-$C_6$ alkyl, hydroxymethyl, acyloxymethyl, and formyl: $R^2$, $R^3$, and $R^4$ are independently hydrogen, hydroxyl, $C_1$-$C_6$ alkyl, $C_2$-$C_6$ alkenyl, $C_2$-$C_6$ alkynyl, $C_1$-$C_6$ alkoxy, halogen, amino, $C_2$-$C_7$ acylamino, $C_1$-$C_6$ alkylamino, $NR^5R^6$ [ in which $R^5$ and $R^6$ are independently $C_1$-$C_6$ alkyl or may form a five-membered or six-membered ring together with a nitrogen atom bonded thereto or further together with an atom of O, S, or N-$R^7$ ($R^7$ being hydrogen, $C_1$-$C_6$ alkyl or an amino-protecting group) as a ring constituent, and a ring-constituting carbon may be substituted by $C_1$-$C_3$ alkyl, amino, or aminomethyl ], phenyl, naphthyl, or pyridyl, or $R^1$ and $R^2$ may form together a bridged structure represented by $-(CH_2)_m$-Z-$(CH_2)_n$- [ in which Z is O, S, CH-$R^8$ ($R^8$ being hydrogen or $C_1$-$C_6$ alkyl), or N-$R^{7'}$ ($R^{7'}$ being hydrogen, $C_1$-$C_6$ alkyl, or an amino-protecting group); and m and n are respectively 0, 1, or 2 provided that m and n are not simultaneously zero.].

**2.** The process for producing the compound and the salt thereof of claim 1, wherein the steric configuration at the 4-position of the compound represented by the general formula (I) is the S-configuration.

**3.** A process for producing a pyranoindolizine derivative represented by the general formula (II):

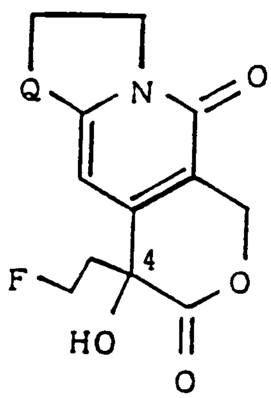

(II)

where Q is C=O (compound $II_1$) or

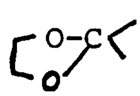

(compound $II_2$), comprising the steps of 2-fluoroethylating the compound of the general formula (IV):

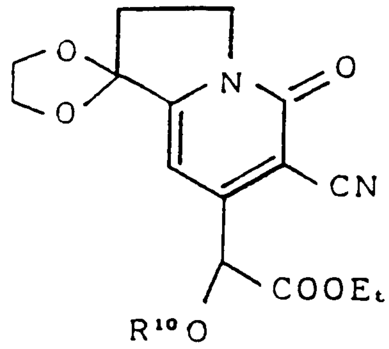

(IV)

where $R^{10}$ is formyl, acetyl, benzoyl, or (R)-1-(p-toluenesulfonyl)prolyl to produce the compound of the general formula ($III_3$):

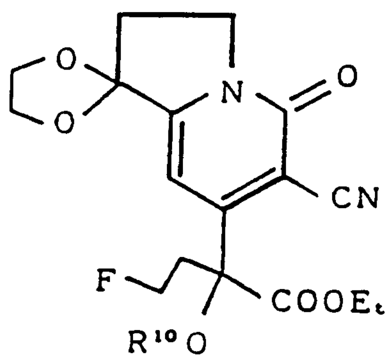

( $III_3$ )

reducing the compound of the general formula ($III_3$) to produce the compound of the general formula ($III_2$) below:

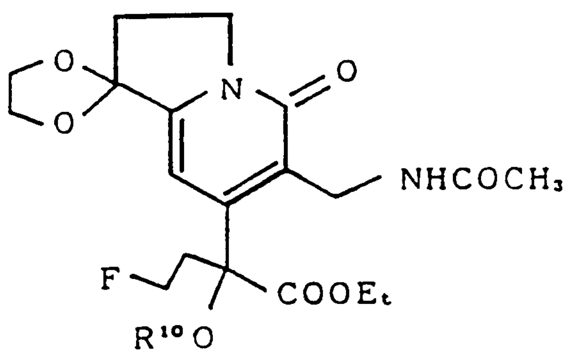

( $III_2$ )

rearranging the compound of the general formula ($III_2$) in the presence of a nitrosation agent to produce the compound of the general formula ($III_1$):

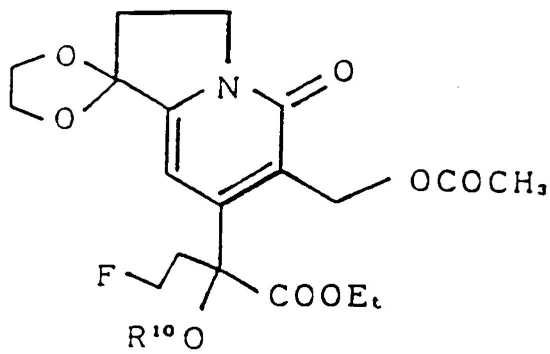

$(III_1)$

causing ring closure of the compound of the general formula $(III_1)$ to produce the compound of the general formula $(II_2)$

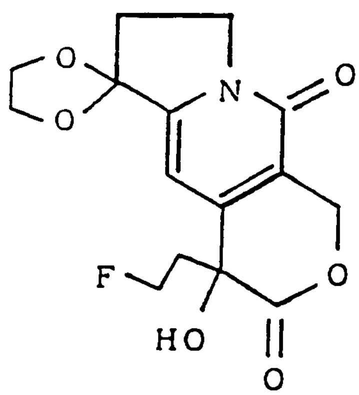

$(II_2)$

and deketalizing the compound of the general formula $(II_2)$, thereby producing the compound of the general formula $(II_1)$.

4. The process for producing a compound $(II_1)$ or $(II_2)$ of claim 3, wherein the steric configuration at the 4-position of the compound represented by the general formula $(II_1)$ or $(II_2)$ is the S-configuration.

5. A process according to claim 3 for producing a pyranoindolizine derivative represented by the general formula (III) below:

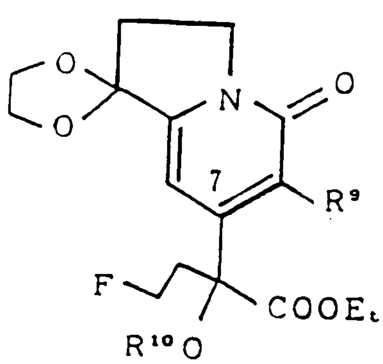

(III)

where $R^9$ is cyano (compound $III_3$), acetylaminomethyl (compound $III_2$) or acetoxymethyl (compound $III_1$); and $R^{10}$ is formyl, acetyl, benzoyl, or (R)-1-(p-toluenesulfonyl)prolyl.

6. The process of claim 5, wherein the steric configuration at the α-position of the acetic acid group at the 7-position of the produced compounds of the general formula (III) is the S-configuration.

**Patentansprüche**

**Patentansprüche für folgende Vertragsstaaten : BE, CH, DE, FR, GB, IT, LI, NL, SE**

1. Verbindung der nachstehenden allgemeinen Formel ( I ) und ein Salz davon

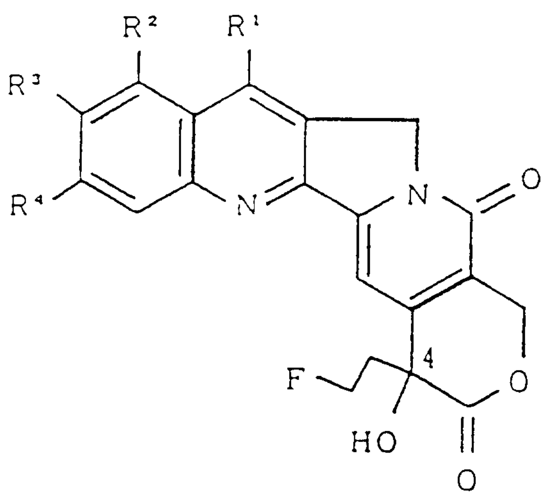

( I )

wobei $R^1$ ein Wasserstoffatom, ein $C_1$-$C_6$-Alkylrest, eine Hydroxymethylgruppe, ein Acyloxymethylrest oder eine Formylgruppe ist; $R^2$, $R^3$ und $R^4$ unabhängig voneinander ein Wasserstoffatom, eine Hydroxylgruppe, ein $C_1$-$C_6$-Alkyl-, $C_2$-$C_6$-Alkenyl-, $C_2$-$C_6$-Alkinyl-, $C_1$-$C_6$-Alkoxyrest, ein Halogenatom, eine Aminogruppe, ein $C_2$-$C_7$-Acylamino-, $C_1$-$C_6$-Alkylaminorest, ein Rest $NR^5R^6$ [ wobei $R^5$ und $R^6$ unabhängig voneinander ein $C_1$-$C_6$-Alkylrest sind oder zusammen mit dem Stickstoffatom, an das sie gebunden sind, einen Fünf- oder Sechsring bilden, der gegebenenfalls ein Sauerstoffatom, ein Schwefelatom oder einen Rest N-$R^7$ ( wobei $R^7$ ein Wasserstoffatom, ein $C_1$-$C_6$-Alkylrest oder eine Aminoschutzgruppe ist ) als Ringbestandteil aufweisen kann und bei dem ein Kohlenstoffatom des Rings mit einem $C_1$-$C_3$-Alkylrest, einer Amino- oder Aminomethylgruppe substituiert sein kann ], eine Phenyl-, Naphthyl-, oder Pyridylgruppe sind oder $R^1$ und $R^2$ zusammen eine überbrückte Struktur $-(CH_2)_m$-Z-$(CH_2)_n$-bilden können [ in der Z ein Sauerstoffatom, ein Schwefelatom, ein Rest CH-$R^8$ ( wobei $R^8$ ein Wasserstoffatom oder ein $C_1$-$C_6$-Alkylrest ist ) oder ein Rest N-$R^{7'}$ ist ( wobei $R^{7'}$ ein Wasserstoffatom, ein $C_1$-$C_6$-Alkylrest oder eine Aminoschutzgruppe ist ) und m bzw. n die Bedeutung 0, 1 oder 2 haben, mit der Maßgabe, daß m und n nicht gleichzeitig Null sind ].

2. Verbindung und Salz nach Anspruch 1, wobei die sterische Konfiguration in 4-Stellung der Verbindung der allgemeinen Formel ( I ) die S-Konfiguration ist.

**3.** Pyranoindolizinderivat der allgemeinen Formel ( II )

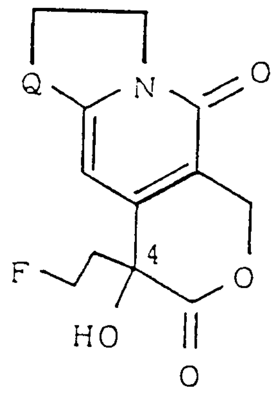

( II )

wobei Q die Bedeutung C=O oder

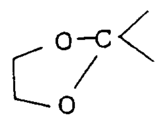

hat.

**4.** Verbindung nach Anspruch 3, in der die sterische Konfiguration in 4-Stellung der Verbindung der allgemeinen Formel ( II ) die S-Konfiguration ist.

**5.** Pyranoindolizinderivat der nachstehenden allgemeinen Formel ( III )

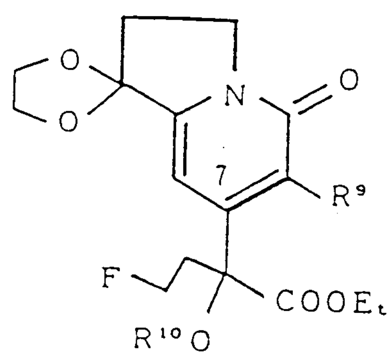

( III )

wobei $R^9$ eine Cyano- Acetylaminomethyl- oder Acetoxymethylgruppe ist; und $R^{10}$ eine Formyl-, Acetyl-, Benzoyl- oder (R)-1-(p-Toluolsulfonyl)prolylgruppe ist.

**6.** Verbindung nach Anspruch 5, wobei die sterische Konfiguration in α-Stellung der Essigsäuregruppe in 7-Stellung der Verbindung der allgemeinen Formel ( III ) die S-Konfiguration ist.

7. Verfahren zur Herstellung eines Pyranoindolizinderivats der allgemeinen Formel ( $II_1$ )

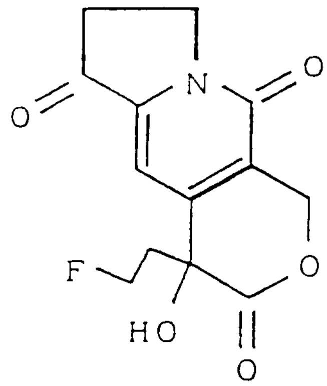

( $II_1$ )

umfassend die Schritte:
2-Fluorethylierung der Verbindung der allgemeinen Formel ( IV )

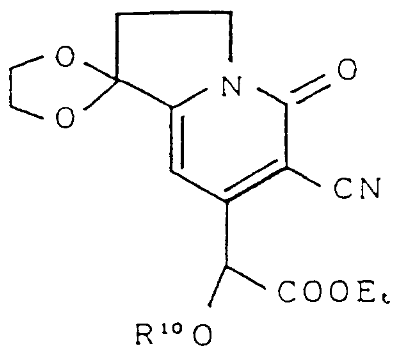

(IV)

wobei $R^{10}$ eine Formyl-, Acetyl-, Benzoyl- oder (R)-1-(p-Toluolsulfonyl)prolylgruppe ist, wobei die Verbindung der allgemeinen Formel ( $III_3$ )

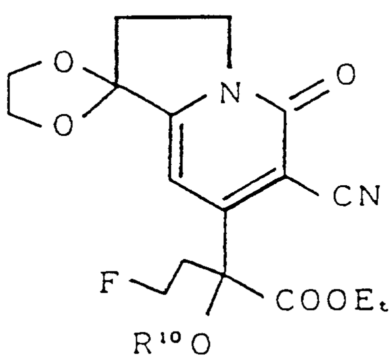

( $III_3$ )

hergestellt wird,
Reduzieren der Verbindung der allgemeinen Formel ( $III_3$ ), wobei die Verbindung der nachstehenden allgemeinen Formel ( $III_2$ )

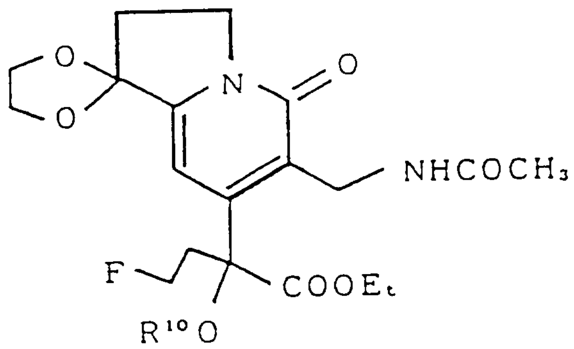

( $III_2$ )

hergestellt wird,
Umlagern der Verbindung der allgemeinen Formel ( $III_2$ ) in Gegenwart eines Nitrosierungsmittels, wobei die Verbindung der allgemeinen Formel ( $III_1$ )

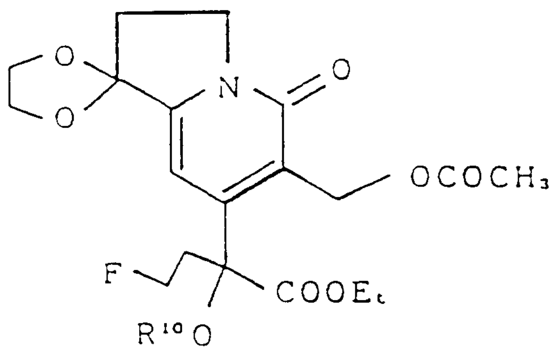

( $III_1$ )

hergestellt wird,
Durchführen des Ringschlusses bei der Verbindung der allgemeinen Formel ( $III_1$ ), wobei die Verbindung der allgemeinen Formel ( $II_2$ )

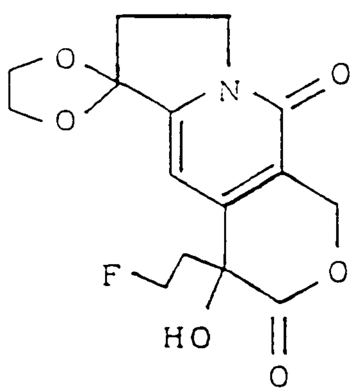

( $II_2$ )

hergestellt wird,
und Entfernen der Ketalgruppe bei der Verbindung der allgemeinen Formel ( $II_2$ ), wobei die Verbindung der allgemeinen Formel ( $II_1$ ) hergestellt wird.

**8.** Verfahren zur Herstellung der Verbindung der allgemeinen Formel ( I ) nach Anspruch 1, umfassend den Schritt der Kondensation der Verbindung der allgemeinen Formel ( $II_1$ )

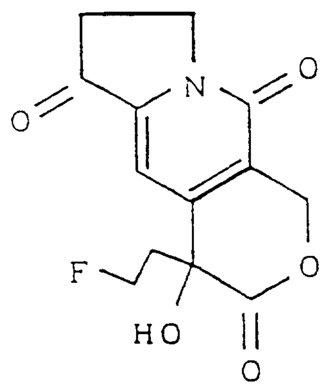

( $II_1$ )

mit der Verbindung der allgemeinen Formel ( V )

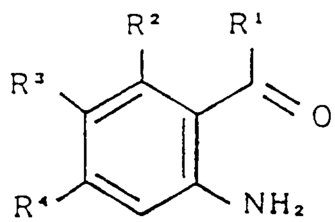

(V)

wobei $R^1$, $R^2$, $R^3$ und $R^4$ wie in Anspruch 1 definiert sind.

**9.** Arzneimittel umfassend eine Verbindung nach Anspruch 1 oder 2 oder ihre Salze und pharmazeutisch verträgliche Verdünnungsmittel und/oder Arzneimittelträger.

**10.** Arzneimittel nach Anspruch 9 mit antineoplastischer Wirksamkeit.

**Patentansprüche für folgenden Vertragsstaat : ES**

1. Verfahren zur Herstellung der Verbindung der allgemeinen Formel ( I )

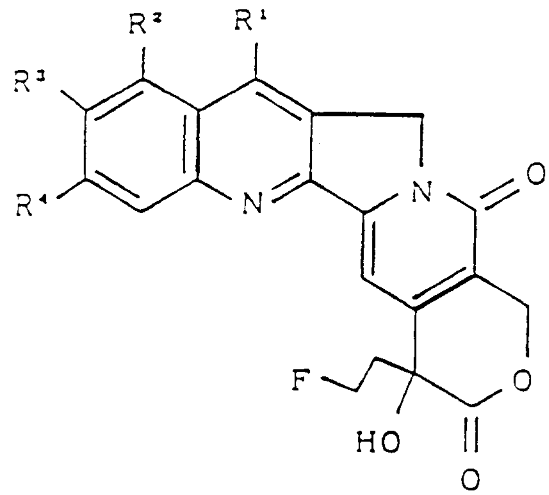

( I )

umfassend den Schritt der Kondensation der Verbindung der allgemeinen Formel ( $II_1$ )

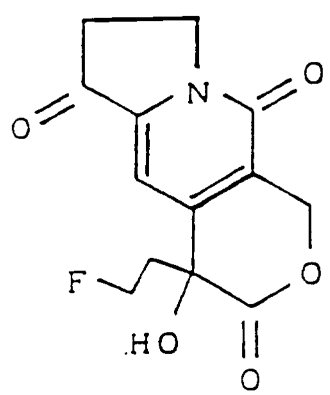

( $II_1$ )

mit der Verbindung der allgemeinen Formel ( V )

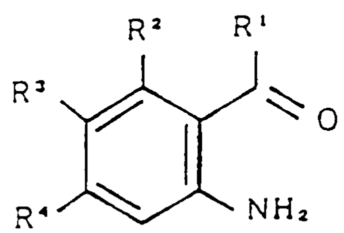

(V)

wobei $R^1$ ein Wasserstoffatom, ein $C_1$-$C_6$-Alkylrest, eine Hydroxymethylgruppe, ein Acyloxymethylrest oder eine Formylgruppe ist; $R^2$, $R^3$ und $R^4$ unabhängig voneinander ein Wasserstoffatom, eine Hydroxylgruppe, ein $C_1$-$C_6$-Alkyl-, $C_2$-$C_6$-Alkenyl-, $C_2$-$C_6$-Alkinyl-, $C_1$-$C_6$-Alkoxyrest, ein Halogenatom, eine Aminogruppe, ein $C_2$-$C_7$-Acylamino-, $C_1$-$C_6$-Alkylaminorest, ein Rest $NR^5R^6$ [ wobei $R^5$ und $R^6$ unabhängig voneinander ein $C_1$-$C_6$-Alkylrest sind

oder zusammen mit dem Stickstoffatom, an das sie gebunden sind, einen Fünf- oder Sechsring bilden, der gegebenenfalls ein Sauerstoffatom, ein Schwefelatom oder einen Rest N-$R^7$ ( wobei $R^7$ ein Wasserstoffatom, ein $C_1$-$C_6$-Alkylrest oder eine Aminoschutzgruppe ist ) als Ringbestandteil aufweisen kann und bei dem ein Kohlenstoffatom des Rings mit einem $C_1$-$C_3$-Alkylrest, einer Amino- oder Aminomethylgruppe substituiert sein kann ], eine Phenyl-, Naphthyl-, oder Pyridylgruppe sind oder $R^1$ und $R^2$ zusammen eine überbrückte Struktur $-(CH_2)_m-Z-(CH_2)_n-$bilden können [ in der Z ein Sauerstoffatom, ein Schwefelatom, ein Rest CH-$R^8$ ( wobei $R^8$ ein Wasserstoffatom oder ein $C_1$-$C_6$-Alkylrest ist ) oder ein Rest N-$R^{7'}$ ist ( wobei $R^{7'}$ ein Wasserstoffatom, ein $C_1$-$C_6$-Alkylrest oder eine Aminoschutzgruppe ist ) und m bzw. n die Bedeutung 0, 1 oder 2 haben, mit der Maßgabe, daß m und n nicht gleichzeitig Null sind ].

**2.** Verfahren zur Herstellung der Verbindung und des Salzes nach Anspruch 1, wobei die sterische Konfiguration in 4-Stellung der Verbindung der allgemeinen Formel ( I ) die S-Konfiguration ist.

**3.** Verfahren zur Herstellung eines Pyranoindolizinderivats der allgemeinen Formel ( II )

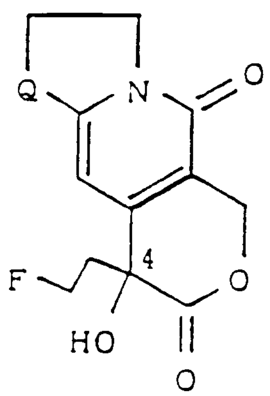

( II )

wobei Q die Bedeutung C=O ( Verbindung $II_1$ ) oder

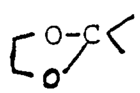

( Verbindung $II_2$ ) hat, umfassend die Schritte:
2-Fluorethylierung dem Verbindung der allgemeinen Formel ( IV )

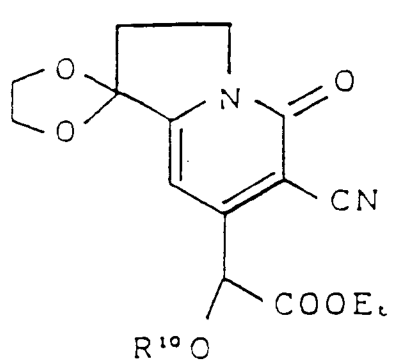

( IV )

wobei $R^{10}$ eine Formyl-, Acetyl-, Benzoyl- oder (R)-1-(p-Toluolsulfonyl)prolylgruppe ist, wobei die Verbindung der allgemeinen Formel ( $III_3$ )

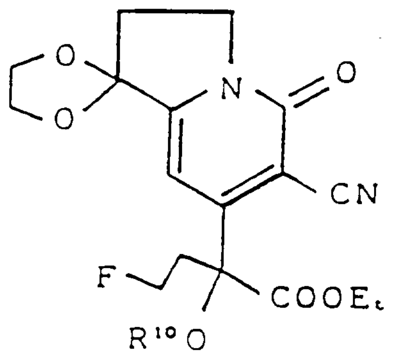

( $III_3$ )

hergestellt wird,
Reduzieren der Verbindung der allgemeinen Formel ( $III_3$ ), wobei die Verbindung der nachstehenden allgemeinen Formel ( $III_2$ )

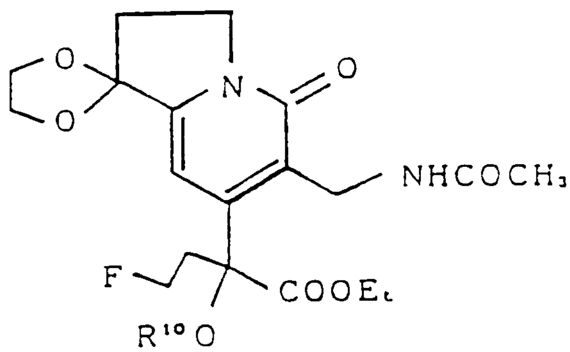

( $III_2$ )

hergestellt wird,
Umlagern der Verbindung der allgemeinen Formel ( $III_2$ ) in Gegenwart eines Nitrosierungsmittels, wobei die Verbindung der allgemeinen Formel ( $III_1$ )

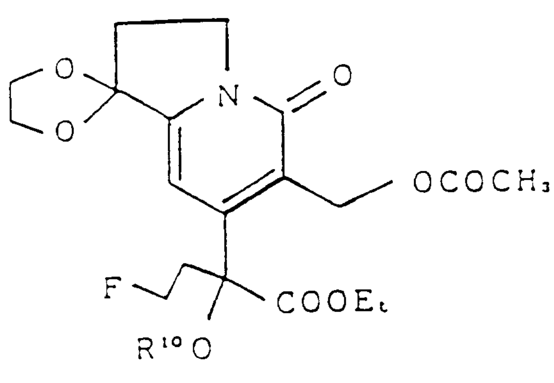

( $III_1$ )

hergestellt wird,
Durchführen des Ringschlusses bei der Verbindung der allgemeinen Formel ( $III_1$ ), wobei die Verbindung der allgemeinen Formel ( $II_2$ )

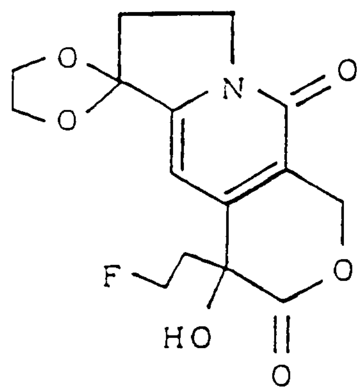

( II₂ )

hergestellt wird,
und Entfernen der Ketalgruppe bei der Verbindung der allgemeinen Formel ( $II_2$ ), wobei die Verbindung der allgemeinen Formel ( $II_1$ ) hergestellt wird.

4. Verfahren zur Herstellung einer Verbindung ( $II_1$ ) oder ( $II_2$ ) nach Anspruch 3, wobei die sterische Konfiguration in 4-Stellung der Verbindung der allgemeinen Formel ( $II_1$ ) oder ( $II_2$ ) die S-Konfiguration ist.

5. Verfahren nach Anspruch 3 zur Herstellung eines Pyranoindolizinderivates der nachstehenden allgemeinen Formel ( III )

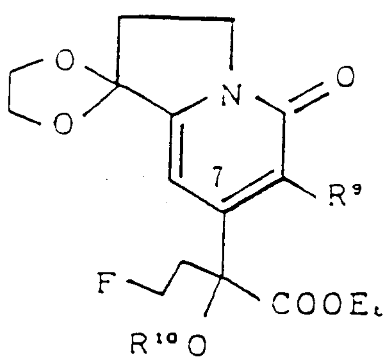

(III)

wobei $R^9$ eine Cyano- ( Verbindung $III_3$ ), Acetylaminomethyl- ( Verbindung $III_2$ ) oder Acetoxymethylgruppe ( Verbindung $III_1$ ) ist; und $R^{10}$ eine Formyl-, Acetyl-, Benzoyl- oder (R)-1-(p-Toluolsulfonyl)prolylgruppe ist.

6. Verfahren nach Anspruch 5, wobei die sterische Konfiguration in $\alpha$-Stellung der Essigsäuregruppe in 7-Stellung der hergestellten Verbindungen der allgemeinen Formel ( III ) die S-Konfiguration ist.

## Revendications

### Revendications pour les Etats contractants suivants : BE, CH, DE, FR, GB, IT, LI, NL, SE

**1.** Composé représenté par la formule générale (I) ci-dessous et un de ses sels:

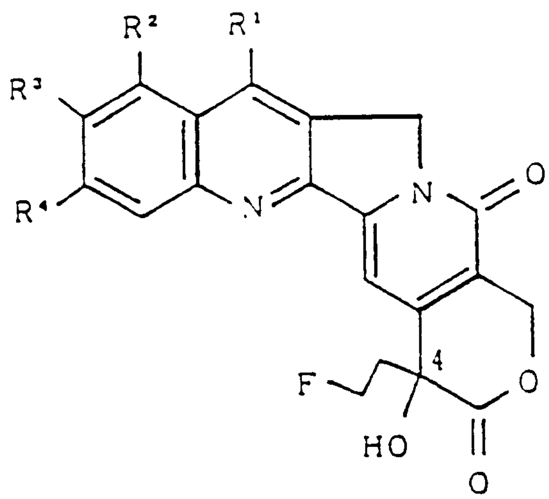

(I)

dans laquelle $R^1$ représente un atome d'hydrogène, un groupe alkyle en $C_1$-$C_6$, un groupe hydroxyméthyle, un groupe acyloxyméthyle et un groupe formyle; $R^2$, $R^3$ et $R^4$ représentent, indépendamment l'un de l'autre, un atome d'hydrogène, un groupe hydroxyle, un groupe alkyle en $C_1$-$C_6$, un groupe alcényle en $C_2$-$C_6$, un groupe alcynyle en $C_2$-$C_6$, un groupe alcoxy en $C_1$-$C_6$, un atome d'halogène, un groupe amino, un groupe acyl(en $C_2$-$C_7$)amino, un groupe alkyl(en $C_1$-$C_6$)amino, $NR^5R^6$ [où $R^5$ et $R^6$ représentent, indépendamment l'un de l'autre, un groupe alkyle en $C_1$-$C_6$, ou bien peuvent former un noyau pentagonal ou hexagonal conjointement avec un atome d'azote qui leur est lié ou encore conjointement avec un atome de O, S, ou encore N-$R^7$ ($R^7$ représentant un atome d'hydrogène, un groupe alkyle en $C_1$-$C_6$ ou un groupe protecteur du groupe amino), comme constituant cyclique, et un atome de carbone de cyclisation peut être substitué par un groupe alkyle en $C_1$-$C_3$, par un groupe amino ou par un groupe aminométhyle], un groupe phényle, un groupe naphtyle ou un groupe pyridyle, ou encore $R^1$ et $R^2$ peuvent former ensemble une structure en pont représentée par $-(CH_2)_m-Z-(CH_2)_n-$ [où Z représente O, S, CH-$R^8$ ($R^8$ représentant un atome d'hydrogène ou un groupe alkyle en $C_1$-$C_6$), ou N-$R^{7'}$ ($R^{7'}$ représentant un atome d'hydrogène, un groupe alkyle en $C_1$-$C_6$ ou un groupe protecteur du groupe amino); et m et n représentent respectivement 0, 1 ou 2 à condition que m et n ne représentent pas simultanément zéro].

**2.** Composé et son sel selon la revendication 1, dans lequel la configuration stérique en position 4 du composé représenté par la formule générale (I) est la configuration S.

**3.** Dérivé de pyranoindolizine représenté par la formule générale (II):

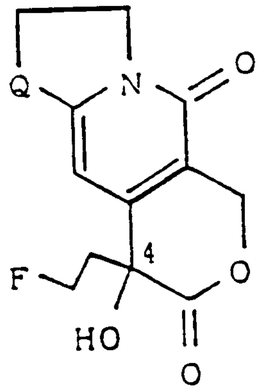

(II)

dans laquelle Q représente C=O ou

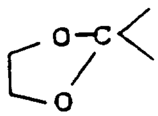

**4.** Composé selon la revendication 3, dans lequel la configuration stérique en position 4 du composé représenté par la formule générale (II) est la configuration S.

**5.** Dérivé de pyranoindolizine représenté par la formule générale (III) ci-dessous:

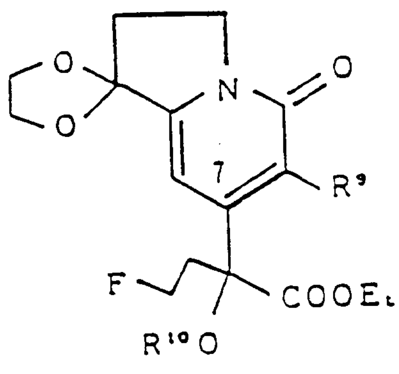

(III)

dans laquelle $R^9$ représente un groupe cyano, un groupe acétylaminométhyle ou un groupe acétoxyméthyle; et $R^{10}$ représente un groupe formyle, un groupe acétyle, un groupe benzoyle ou un groupe (R)-1-(p-toluènesulfonyl)prolyle.

**6.** Composé selon la revendication 5, dans lequel la configuration stérique en position α du groupe d'acide acétique en position 7 du composé de formule générale (III) est la configuration S.

**7.** Procédé pour préparer un dérivé de pyranoindolizine représenté par la formule générale ($II_1$):

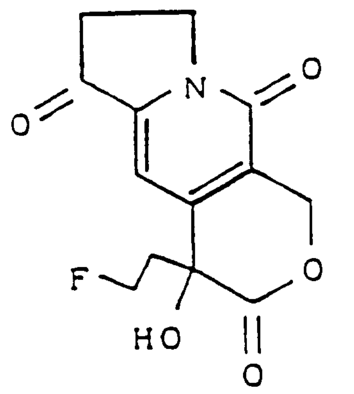

( $II_1$ )

comprenant les étapes consistant à soumettre le composé de formule générale (IV) à une 2-fluoréthylation:

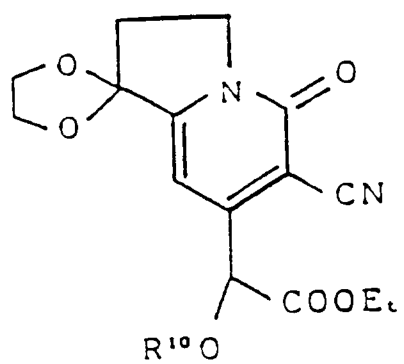

(IV)

où $R^{10}$ représente un groupe formyle, un groupe acétyle, un groupe benzoyle ou un groupe (R)-1-(p-toluènesulfonyl)prolyle pour obtenir le composé de formule générale ($III_3$):

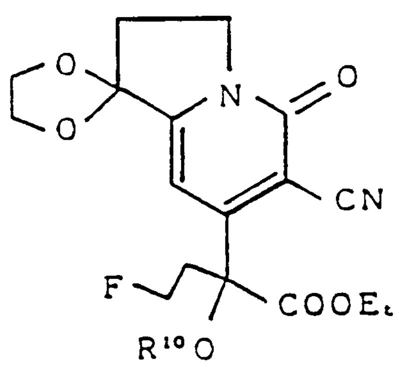

( $III_3$ )

soumettre le composé de formule générale ($III_3$) à une réduction pour obtenir le composé de formule générale ($III_2$) ci-dessous:

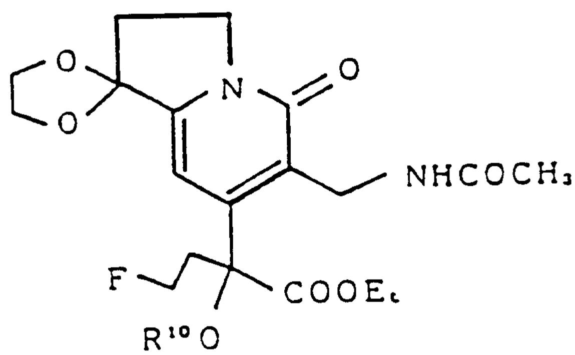

$(III_2)$

soumettre le composé de formule générale ($III_2$) à un réarrangement en présence d'un agent de nitrosation pour obtenir le composé de formule générale ($III_1$):

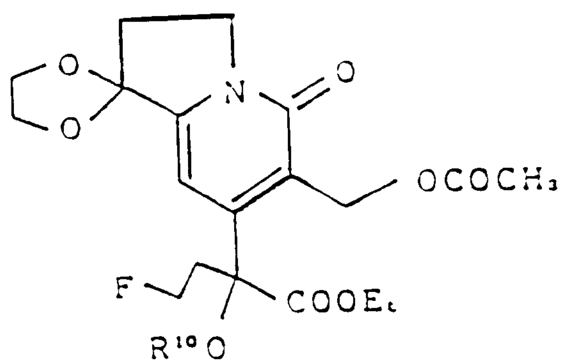

$(III_1)$

soumettre le composé de formule générale ($III_1$) à une cyclisation pour obtenir le composé de formule générale ($II_2$):

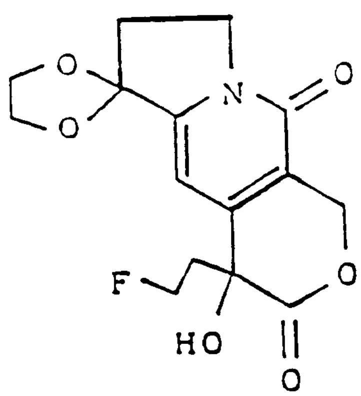

$(II_2)$

et soumettre le composé de formule générale ($II_2$) à une décétalisation pour obtenir le composé de formule générale ($II_1$).

**8.** Procédé pour obtenir le composé de formule générale (I) selon la revendication 1 comprenant l'étape consistant à condenser le composé de formule générale ($II_1$)

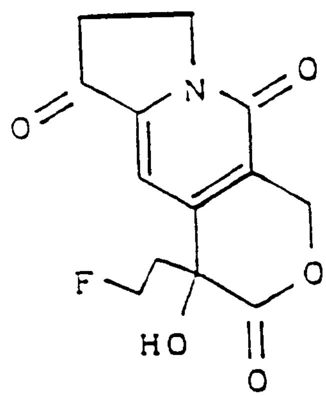

( II₁ )

avec le composé de formule générale (V)

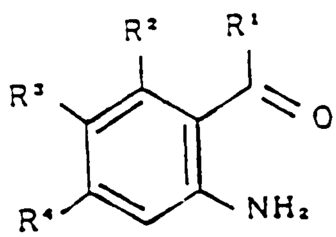

(V)

dans laquelle $R^1$, $R^2$, $R^3$ et $R^4$ sont tels que définis à la revendication 1.

9. Compositions pharmaceutiques comprenant un composé selon l'une quelconque des revendications 1 ou 2 ou leurs sels, ainsi que des diluants et/ou des supports pharmaceutiquement acceptables.

10. Composition pharmaceutique selon la revendication 9, possédant une activité antinéoplasique.

**Revendications pour l'Etat contractant suivant : ES**

1. Procédé pour obtenir le composé de formule générale (I):

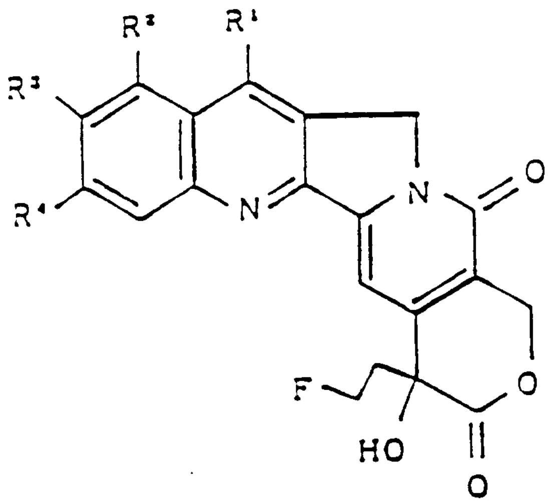

(I)

comprenant l'étape consistant à condenser le composé de formule générale ($II_1$):

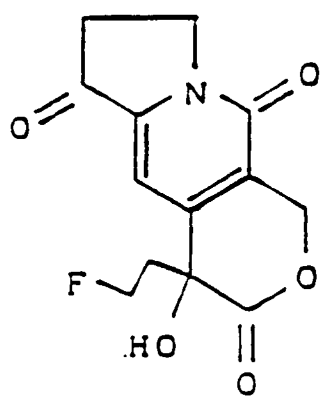

($II_1$)

avec le composé de formule générale (V):

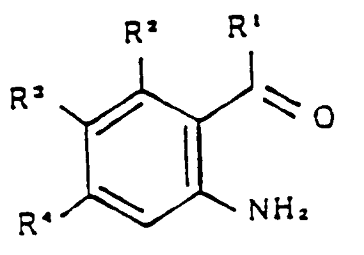

(V)

dans laquelle $R^1$ représente un atome d'hydrogène, un groupe alkyle en $C_1$-$C_6$, un groupe hydroxyméthyle, un groupe acyloxyméthyle et un groupe formyle; $R^2$, $R^3$ et $R^4$ représentent, indépendamment l'un de l'autre, un atome d'hydrogène, un groupe hydroxyle, un groupe alkyle en $C_1$-$C_6$, un groupe alcényle en $C_2$-$C_6$, un groupe alcynyle en $C_2$-$C_6$, un groupe alcoxy en $C_1$-$C_6$, un atome d'halogène, un groupe amino, un groupe acyl(en $C_2$-$C_7$)amino, un groupe alkyl(en $C_1$-$C_6$)amino, $NR^5R^6$ [où $R^5$ et $R^6$ représentent, indépendamment l'un de l'autre, un groupe alkyle

en $C_1$-$C_6$, ou bien peuvent former un noyau pentagonal ou hexagonal conjointement avec un atome d'azote qui leur est lié ou encore conjointement avec un atome de O, S, ou encore N-$R^7$ ($R^7$ représentant un atome d'hydrogène, un groupe alkyle en $C_1$-$C_6$ ou un groupe protecteur du groupe amino), comme constituant cyclique, et un atome de carbone de cyclisation peut être substitué par un groupe alkyle en $C_1$-$C_3$, par un groupe amino ou par un groupe aminométhyle], un groupe phényle, un groupe naphtyle ou un groupe pyridyle, ou encore $R^1$ et $R^2$ peuvent former ensemble une structure en pont représentée par $-(CH_2)_m$-Z-$(CH_2)_n$- [où Z représente O, S, CH-$R^8$ ($R^8$ représentant un atome d'hydrogène ou un groupe alkyle en $C_1$-$C_6$), ou N-$R^{7'}$ ($R^{7'}$ représentant un atome d'hydrogène, un groupe alkyle en $C_1$-$C_6$ ou un groupe protecteur du groupe amino); et m et n représentent respectivement 0, 1 ou 2 à condition que m et n ne représentent pas simultanément zéro].

2. Procédé pour obtenir le composé et son sel selon la revendication 1, dans lequel la configuration stérique en position 4 du composé représenté par la formule générale (I) est la configuration S.

3. Procédé pour obtenir un dérivé de pyranoindolizine représenté par la formule générale (II):

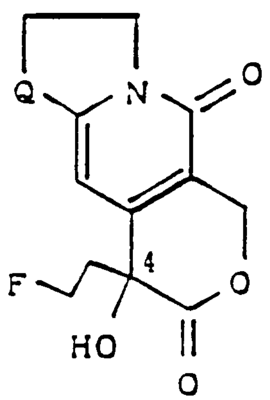

(II)

dans laquelle Q représente C=O (composé $II_1$) ou (composé $II_2$),

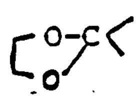

comprenant les étapes consistant à soumettre le composé de formule générale (IV) à une 2-fluoréthylation:

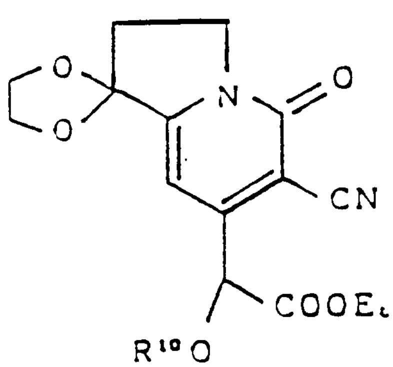

(IV)

où $R^{10}$ représente un groupe formyle, un groupe acétyle, un groupe benzoyle ou un groupe (R)-1-(p-toluènesulfonyl)prolyle pour obtenir le composé de formule générale ($III_3$):

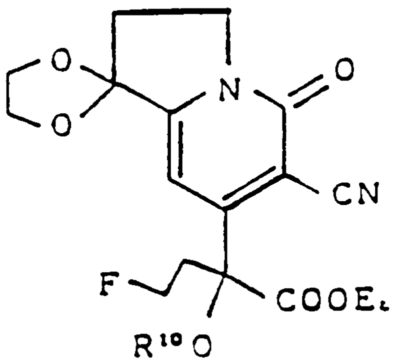

( III₃ )

soumettre le composé de formule générale ($III_3$) à une réduction pour obtenir le composé de formule générale ($III_2$) ci-dessous:

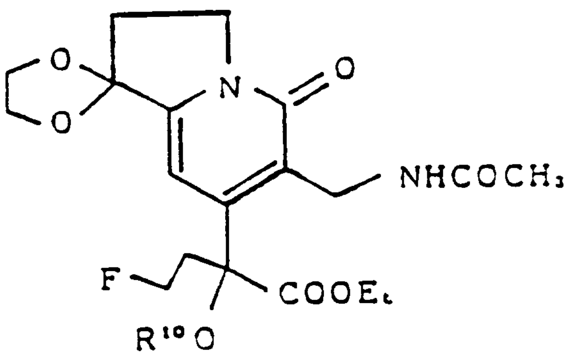

( III₂ )

soumettre le composé de formule générale ($III_2$) à un réarrangement en présence d'un agent de nitrosation pour obtenir le composé de formule générale ($III_1$):

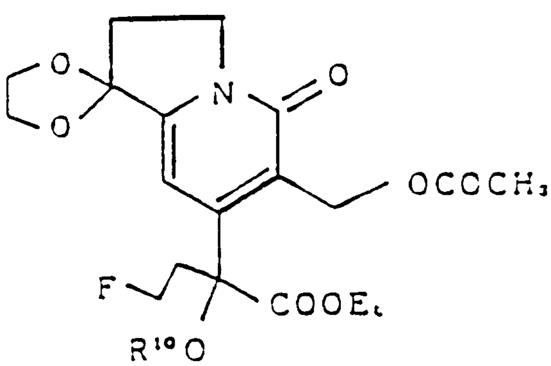

( III₁ )

soumettre le composé de formule générale ($III_1$) à une cyclisation pour obtenir le composé de formule générale ($II_2$):

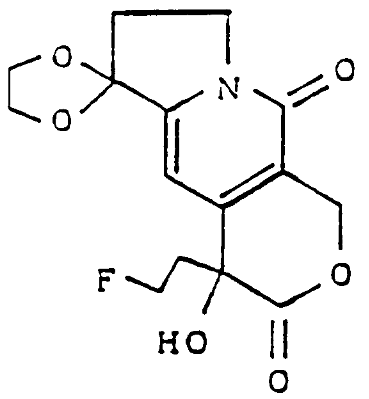

( II₂ )

et soumettre le composé de formule générale ($II_2$) à une décétalisation pour obtenir le composé de formule générale ($II_1$).

4. Procédé pour obtenir un composé ($II_1$) ou ($II_2$) selon la revendication 3, dans lequel la configuration stérique en position 4 du composé représenté par la formule ($II_1$) ou ($II_2$) est la configuration S.

5. Procédé selon la revendication 3, pour obtenir un dérivé de pyranoindolizine représenté par la formule générale (III) ci-dessous:

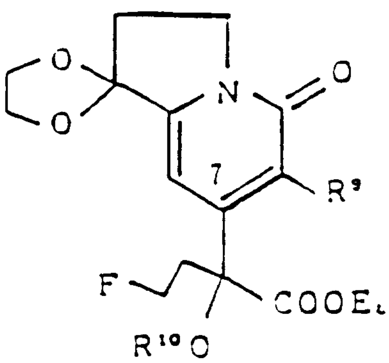

(III)

dans laquelle $R^9$ représente un groupe cyano (composé $III_3$), un groupe acétylaminométhyle (composé $III_2$) au un groupe acétoxyméthyle (composé $III_1$); et $R^{10}$ représente un groupe formyle, un groupe acétyle, un groupe benzoyle ou un groupe (R)-1-(p-toluènesulfonyl)prolyle.

6. Procédé selon la revendication 5, dans lequel la configuration stérique en position $\alpha$ du groupe d'acide acétique en position 7 des composés obtenus répondant à la formule générale (III) est la configuration S.